(19) Europäisches Patentamt
European Patent Office
Office européen des brevets

(11) EP 3 129 419 B1

(12) **EUROPÄISCHE PATENTSCHRIFT**

(45) Veröffentlichungstag und Bekanntmachung des Hinweises auf die Patenterteilung:
**19.09.2018 Patentblatt 2018/38**

(21) Anmeldenummer: **15712975.0**

(22) Anmeldetag: **01.04.2015**

(51) Int Cl.:
***C08G 2/38*** *(2006.01)* ***C08G 64/18*** *(2006.01)*
***C08G 64/34*** *(2006.01)* ***C08G 64/42*** *(2006.01)*
***C08G 18/44*** *(2006.01)*

(86) Internationale Anmeldenummer:
**PCT/EP2015/057209**

(87) Internationale Veröffentlichungsnummer:
**WO 2015/155094 (15.10.2015 Gazette 2015/41)**

(54) **VERFAHREN ZUR HERSTELLUNG VON POLYOXYMETHYLEN-BLOCKCOPOLYMEREN**

METHOD FOR THE PREPARATION OF POLYOXYMETHYLENE BLOCK COPOLYMERS

PROCÉDÉ DE FABRICATION DE COPOLYMÈRES SÉQUENCÉS DE POLYOXYMÉTHYLÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.04.2014 EP 14163744**
**23.02.2015 EP 15156102**

(43) Veröffentlichungstag der Anmeldung:
**15.02.2017 Patentblatt 2017/07**

(73) Patentinhaber: **Covestro Deutschland AG**
**51373 Leverkusen (DE)**

(72) Erfinder:
- **PECKERMANN, Ilja**
**50735 Köln (DE)**
- **WOLF, Aurel**
**42489 Wülfrath (DE)**
- **LANGANKE, Jens**
**53894 Mechernich (DE)**
- **GÜRTLER, Christoph**
**50735 Köln (DE)**
- **HOFMANN, Jörg**
**47800 Krefeld (DE)**

(74) Vertreter: **Levpat**
**c/o Covestro AG**
**Gebäude 4825**
**51365 Leverkusen (DE)**

(56) Entgegenhaltungen:
**EP-A1- 1 870 425**
**WO-A1-2004/096746**
**WO-A1-2012/091968**
**JP-A- 2007 211 082**
**US-A1- 2006 205 915**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 129 419 B1

## Beschreibung

Die vorliegende Erfindung beschreibt ein Verfahren zur Herstellung von Polyoxymethylen-Blockcopolymeren. Sie betrifft weiterhin durch ein solches Verfahren erhältliche Polyoxymethylen-Blockcopolymere sowie deren Verwendung.

Blockcopolymere enthaltend Polyoxymethylen-Einheiten neben anderen Polymerisat- und Polykondensat-Einheiten werden beispielsweise in JP 2007 211082 A, WO 2004/096746 A1, GB 807589, EP 1 418 190 A1, US 3,754,053, US 3,575,930, US 2002/0016395, und JP 04-306215 beschrieben.

US 3,575,930 beschreibt die Reaktion von Dihydroxy-terminiertem Paraformaldehyd HO-$(CH_2O)_n$-H mit n = 2-64 mit Diisocyanaten zu Isocyanat-terminierten Polyoxymethylenpolymeren, welche in der Reaktion mit Diolen zu Polyurethanverbindungen umgesetzt werden können.

JP 2007 211082 A beschreibt die Umsetzung von Polyoxyalkylen-Polyolen mit einem Äquivalentgewicht von ≥ 2500 mit Formaldehyd, Formaldehyd-Oligomeren oder Formaldehyd-Polymeren zu Polyoxymethylen-Polyoxyalkylen-Blockcopolymeren unter Anwendung von anionischen oder kationischen Polymerisationskatalysatoren. Die eingesetzten hochmolekularen Polyoxyalkylen-Polyol-Starter mit niedriger Polydispersität werden über Doppelmetallcyanid (DMC)-Katalyse hergestellt. Aufgrund des hohen Molekulargewichtes der Polyoxyalkylen-Polyole weisen die erhaltenen Polyoxymethylen-Polyoxyalkylen-Blockcopolymere ein Molekulargewicht von wenigstens > 5000 g/mol auf und sind daher weniger breit als Polyurethan-Baustein einsetzbar. Weiterhin macht die direkte Umsetzung der Polyoxyalkylen-Polyole mit den Polyoxymethylen-Polymeren über ein Schmelz-Knet-Verfahren den Einsatz von hohen Temperaturen und entsprechenden spezifischen Hochviskosapparaten (Extruder, Kneter, etc.) notwendig.

US 3,754,053 beschreibt Polyoxymethylen-Polyoxyalkylen-Blockcopolymere mit einem Molekulargewicht ≥ 10 000 g/mol. Zur Herstellung von Copolymeren mit einem inneren Polyoxymethylen-Block wird in einem erstem Schritt Trioxan zu einem Polyoxymethylen-Präpolymer umgesetzt und dieses dann in Gegenwart von z.B. NaOH als Polymerisationskatalysator mit Alkylenoxiden umgesetzt. Auch hier sind die beschriebenen Polymere aufgrund ihres hohen Molekulargewichts für Anwendungen als Polyurethanbaustein weniger gut geeignet.

WO 2004/096746 A1 und US 2006/0205915 A1 offenbaren die Reaktion von Formaldehyd-Oligomeren mit Alkylenoxiden und/oder Isocyanaten. Bei dieser Methode werden über den beschriebenen Einsatz von Formaldehyd-Oligomeren HO-$(CH_2O)_n$-H Polyoxymethylen-Blockcopolymere mit einer relativ engen Molmassenverteilung von n = 2-19 erhalten, wobei für die Bereitstellung der Formaldehyd-Oligomere ausgehend von wässriger Formalinlösung ein zusätzlicher thermischer Abtrenn-Verfahrensschritt erforderlich ist. Die erhaltenen Formaldehyd-Oligomer-Lösungen sind hierbei nicht lagerstabil, so dass diese anschließend sofort weiterverarbeitet werden müssen. Außerdem werden in diesen Anmeldungen keine differenzierten Aktivierungsbedingungen, wie beispielsweise der Aktivierungstemperatur, der verwendeten Alkoxylierungskatalysatoren offenbart, welche auch aus sicherheitstechnischen sowie qualitätsrelevanten Gesichtspunkten für eine mögliche großtechnische Anwendung durch undefinierte Temperaturspitzen während des exothermen Polymerisationsprozesses (22.7 kcal/mol PO from M. Ionesco; Chemistry and Technology of Polyols for Polyurethanes, Rapra Techn. Ltd., 2005) nachteilig sind. Weiterhin sind über diese Methode nur Blockcopolymere mit sehr kurzen Formaldehyd-Blöcken zugänglich.

In EP 1 870 425 A1 wird ein Verfahren zu Herstellung von Polyoxyalkylen-haltigen Polyolen durch Kondensation von substituierten oder unsubstituierten Phenolstrukturen mit Formaldehyden und/oder anderen substituierten Alkanalstrukturen offenbart. Die resultierenden Phenol-Formaldehyd-Kondensate werden hierbei als Polyolstarter für die Alkoxylierung verwendet, wobei innerhalb dieser Starterverbindungen keine Oxymethylen-Wiederholungseinheiten ausgebildet werden. Weiterhin unterscheiden sich resultierenden Eigenschaften der alkoxylierten, Aromatenhaltigen Poylole aufgrund der unterschiedlichen chemischen Struktur grundlegend von aliphatischen Polyolstrukturen.

In WO2012/091968 A1 wird ein Verfahren zur Herstellung Polyetherolen durch Polymerisation von Alkylenoxiden an Starterverbindungen mithilfe von DMC Katalysatoren beansprucht. Hierbei werden als Formaldehyd-assoziierte Strukturen oligomere Phenol-Formaldehyde Kondensate als entsprechende Starter offenbart, die sich strukturell grundlegend von der Polyoxymethylen-Starterstruktur unterscheidet.

Ausgehend vom Stand der Technik ergab sich daher die Aufgabe ein einfaches und wirtschaftlich vorteilhaftes Verfahren zur Herstellung von Polyoxymethylen-Blockcopolymeren auf Basis von oligomeren und polymeren Formen des Formaldehyds als Startersubstanz zur Verfügung zu stellen, mit dem die sich aus dem Stand der Technik ergebenden Probleme überwunden werden können.

Hierbei sollte die Spaltung der eingesetzten Formaldehyd-Starterverbindungen in kleinere Polymere, Oligomere und Monomere, die Bildung von Neben- und Zersetzungsprodukten möglichst vermieden werden sowie die Verfahrenssicherheit erhöht werden. Weiterhin sollten hierüber stabile niedermolekulare Polyoxymethylen-Blockcopolymere erhältlich sein, welche Hydroxy-funktionalisierte Endgruppen aufweisen und sich für die Reaktion mit Di- oder Polyisocyanaten zur Herstellung von Polyurethanen eignen. Das erfindungsgemäße Verfahren sollte vorzugsweise auch geeignet sein, Polyoxymethylen-Polyoxyalkylencarbonat-Blockcopolymere zugänglich zu machen, wobei ein hoher Gehalt an eingebautem $CO_2$ erreicht wird.

Erfindungsgemäß gelöst wird diese Aufgabe durch ein Verfahren zur Herstellung von Polyoxymethylen-Blockcopolymeren durch katalytische Anlagerung von Alkylenoxiden sowie gegebenenfalls weiteren Comonomeren an wenigstens eine polymere Formaldehyd-Starterverbindung, welche wenigstens eine terminale Hydroxylgruppe aufweist, in Gegenwart eines Doppelmetallcyanid (DMC)-Katalysators, wobei

(i) in einem ersten Schritt der DMC-Katalysator in Gegenwart der polymeren Formaldehyd-Starterverbindung aktiviert wird, wobei zur Aktivierung des DMC-Katalysators eine Teilmenge (bezogen auf die Gesamtmenge der bei der Aktivierung und Polymerisation eingesetzten Menge an Alkylenoxiden) von einem oder mehreren Alkylenoxiden zugesetzt wird ("Aktivierung"),

(ii) in einem zweiten Schritt ein oder mehrere Alkylenoxide sowie gegebenenfalls weitere Comonomere zu der aus Schritt (i) resultierenden Mischung zugesetzt werden, wobei die in Schritt (ii) eingesetzten Alkylenoxide gleich oder verschieden sein können von den bei Schritt (i) eingesetzten Alkylenoxiden ("Polymerisation"),

und wobei die Aktivierung des DMC-Katalysators im ersten Schritt (i) bei einer Aktivierungstemperatur ($T_{act}$) von 20 bis 120 °C erfolgt.

Weitere Gegenstände der vorliegenden Erfindung sind die über das erfindungsgemäße Verfahren erhältlichen Polyoxymethylen-Blockcopolymere, deren Verwendung sowie Polyurethan-Polymere enthaltend die erfindungsgemäßen Polyoxymethylen-Blockcopolymere.

Die Verwendung des Worts ein im Zusammenhang mit zählbaren Größen ist hierbei und im Folgenden nur dann als Zahlwort zu verstehen, wenn dies aus dem Zusammenhang hervorgeht (bspw. durch die Formulierung "genau ein"). Ansonsten umfassen Ausdrücke wie "ein Alkylenoxid", "eine polymere Formaldehyd-Starterverbindung" etc. immer auch solche Ausführungsformen, in denen zwei oder mehr Alkylenoxide, zwei oder mehr polymere Formaldehyd-Starterverbindungen etc. eingesetzt werden.

Nachfolgend wird die Erfindung im Detail erläutert. Verschiedene Ausführungsformen sind dabei beliebig miteinander kombinierbar, solange sich für den Fachmann aus dem Kontext nicht eindeutig das Gegenteil ergibt.

Polyoxymethylen-Blockcopolymere im Sinne der Erfindung bezeichnen polymere Verbindungen, die mindestens einen Polyoxymethylen-Block sowie mindestens einen zusätzlichen oligomeren Block (z.B. Polyoxyalkylen- bzw. Polyoxyalkylencarbonat-Blöcke) enthalten und vorzugsweise ein im vierstelligen Bereich liegendes Molekulargewicht nicht überschreiten.

Die erhaltenen Polyoxymethylen-Blockcopolymere bieten gegenüber existierenden Polymeren eine Reihe von Vorteilen. So können bestimmte physikalische Eigenschaften wie Glasübergangstemperaturen, Schmelzbereiche, Viskositäten und Löslichkeiten, etc. über die Länge der Polyoxymethylen-Blöcke im Verhältnis zu den anderen oligomeren Blöcken gezielt angesteuert werden.

Gegenüber Polyoxymethylen-Homopolymeren gleichen Molekulargewichts ist die Teilkristallinität in den erfindungsgemäßen Polyoxymethylen-Blockcopolymeren typischerweise herabgesetzt, was üblicherweise ebenfalls zu einer Herabsetzung von Glasübergangstemperaturen, Schmelzpunkten und Viskositäten, etc. führt. Die Anwesenheit von zusätzlichen Polyoxyalkylenblöcken führt weiterhin typischerweise zu einer deutlichen Erhöhung der chemischen und thermischen Stabilität. Zudem weisen die erhaltenen Polyoxymethylen-Blockcopolymere im Allgemeinen gute Löslichkeiten in diversen Lösungsmitteln auf, sind zumeist leicht und ohne Massenverlust aufschmelzbar oder liegen schon bei niedrigen Temperaturen im flüssigen Zustand vor. Gegenüber Polyoxymethylen-Homopolymeren zeigen die Polyoxymethylen-Blockcopolymere somit eine deutlich bessere Verarbeitbarkeit.

Gegenüber Polyetherpolyolen gleichen Molekulargewichts ist der Anteil an PolyoxyalkylenEinheiten, die aus den entsprechenden Alkylenoxiden hergestellt werden, um den Polyoxymethylen-Anteil verringert, was zu einer vorteilhaften Wirtschaftlichkeit des Produktes beiträgt. Diverse physikalische Eigenschaften wie Glasübergangstemperaturen, Schmelzbereiche, Viskositäten, Löslichkeit, etc. können für ein gegebenes Molekulargewicht über die Länge der Polyoxymethylen-Blöcke im Verhältnis zu den Polyoxyalkylen-Blöcken, sowie über das Molekulargewicht der eingesetzten polymeren Formaldehyd-Starterverbindung (Polyoxymethylen-Block), gezielt angesteuert werden. Die synthetisch variable Molekülstruktur der erhaltenen Polyoxymethylen-Blockcopolymere ermöglicht zudem die Schaffung maßgeschneiderter "hart-weich"-Segmente auf molekularer Ebene. Gegenüber statistischen Polyoxymethylen-Polyoxyalkylen-Copolymeren weisen die erhaltenen Polyoxymethylen-Blockcopolymere aufgrund ihrer Segmentstruktur eine höhere innere Ordnung auf.

Dies kann in vorteilhaften physikalischen Eigenschaften insbesondere von Folgeprodukte dieser Polymere resultieren und somit neue Anwendungen ermöglichen.

Als polymere Formaldehyd-Starterverbindung für das erfindungsgemäße Verfahren eignen sich grundsätzlich solche oligomeren und polymeren Formen des Formaldehyds, welche wenigstens eine terminale Hydroxylgruppe zur Reaktion mit den Alkylenoxiden und den gegebenenfalls weiteren Comonomeren aufweisen. Unter dem Begriff "terminale Hydroxylgruppe" wird erfindungsgemäß insbesondere eine terminale Halbacetal-Funktionalität verstanden, welche sich

als Strukturmerkmal über die Polymerisation des Formaldehyds ergibt. Beispielsweise können die Starterverbindungen Oligomere und Polymere des Formaldehyds der allgemeinen Formel HO-$(CH_2O)_n$-H sein, wobei n für eine ganze Zahl ≥ 2 steht und wobei polymeres Formaldehyd typischerweise n > 8 Wiederholungseinheiten aufweist.

Für das erfindungsgemäße Verfahren geeignete polymere Formaldehyd-Starterverbindungen weisen im Allgemeinen Molmassen von 62 bis 30000 g/mol, bevorzugt von 62 bis 12000 g/mol, besonders bevorzugt von 242 bis 6000 g/mol und ganz besonders bevorzugt von 242 bis 3000 g/mol auf und umfassen von 2 bis 1000, bevorzugt von 2 bis 400, besonders bevorzugt von 8 bis 200 und ganz besonders bevorzugt von 8 bis 100 Oxymethylen-Wiederholungseinheiten. Die im erfindungsgemäßen Verfahren eingesetzten Starterverbindungen haben typischerweise eine Funktionalität (F) von 1 bis 3, in bestimmten Fällen können diese aber auch höherfunktionell sein, also eine Funktionalität > 3 besitzen. Bevorzugt werden im erfindungsgemäßen Verfahren offenkettige polymere Formaldehyd-Starterverbindungen mit terminalen Hydroxylgruppen eingesetzt, welche eine Funktionalität von 1 bis 10, bevorzugt von 1 bis 5, besonders bevorzugt von 2 bis 3 haben. Ganz besonders bevorzugt werden im erfindungsgemäßen Verfahren lineare polymere Formaldehyd-Starterverbindungen eingesetzt, welche eine Funktionalität von 2 aufweisen. Die Funktionalität F entspricht der Anzahl an OH-Endgruppen pro Molekül.

Die Herstellung der polymeren Formaldehyd-Starterverbindungen, welche für das erfindungsgemäße Verfahren eingesetzt werden, kann nach bekannten Verfahren erfolgen (vgl. z.B. M. Haubs et. al., 2012, Polyoxymethylenes, Ullmann's Encyclopedia of Industrial Chemistry; G. Reus *et. al.,* 2012, Formaldehyde, *ibid*). Die Formaldehyd-Starterverbindungen können im erfindungsgemäßen Verfahren grundsätzlich auch in Form eines Copolymers eingesetzt werden, wobei als Comonomere neben Formaldehyd beispielsweise 1,4-Dioxan, oder 1,3-Dioxolan einpolymerisiert sind. Weitere geeignete Formaldehyd-Copolymere für das erfindungsgemäße Verfahren sind Copolymere des Formaldehyds sowie des Trioxans mit cyclischen und/oder linearen Formalen, wie beispielsweise Butandiolformal, oder Epoxiden. Es ist ebenfalls denkbar, dass als Comonomere höhere homologe Aldehyde, wie beispielweise Acetaldehyd, Propionaldehyd, etc., in das Formaldehyd-Polymer eingebaut sind. Ebenfalls ist es denkbar, dass erfindungsgemäße Formaldehyd-Starterverbindungen wiederum ausgehend von H-funktionellen Starterverbindungen hergestellt werden, insbesondere lassen sich hierbei durch den Einsatz von mehrwertigen Starterverbindungen polymere Formaldehyd-Starterverbindungen mit einer Hydroxy-Endgruppen-Funktionalität F > 2 erhalten (vgl. z.B. WO 1981001712 A1, Bull. Chem. Soc. J., 1994, 67, 2560-2566, US 3436375, JP 03263454, JP 2928823).

Für das erfindungsgemäße Verfahren können auch Gemische unterschiedlicher polymerer Formaldehyd-Starterverbindungen oder Gemische mit anderen H-funktionellen Starterverbindungen eingesetzt werden. Als geeignete H-funktionelle Startersubstanz ("Starter") können Verbindungen mit für die Alkoxylierung aktiven H-Atomen eingesetzt werden, die eine Molmasse von 18 bis 4500 g/mol, bevorzugt von 62 bis 2500 g/mol und besonders bevorzugt von 62 bis 1000 g/mol aufweisen. Für die Alkoxylierung aktive Gruppen mit aktiven H-Atomen sind beispielsweise -OH, $-NH_2$ (primäre Amine), -NH- (sekundäre Amine), -SH und $-CO_2H$, bevorzugt sind -OH und $-NH_2$, besonders bevorzugt ist -OH. Als H-funktionelle Startersubstanz wird beispielsweise eine oder mehrere Verbindungen ausgewählt aus der Gruppe bestehend aus ein- oder mehrwertigen Alkoholen, mehrwertigen Aminen, mehrwertigen Thiolen, Aminoalkohole, Thioalkohole, Hydroxyester, Polyetherpolyole, Polyesterpolyole, Polyesteretherpolyole, Polyethercarbonatpolyole, Polycarbonatpolyole, Polycarbonate, Polyethylenimine, Polyetheramine, Polytetrahydrofurane (z. B. PolyTHF® der BASF), Polytetrahydrofuranamine, Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und $C_1$-$C_{24}$ Alkyl-Fettsäureester, die im Mittel mindestens 2 OH-Gruppen pro Molekül enthalten, eingesetzt.

Bekanntlich polymerisiert Formaldehyd bereits durch die Gegenwart geringer Wasserspuren. In wässriger Lösung bildet sich daher in Abhängigkeit von Konzentration und Temperatur der Lösung ein Gemisch von Oligomeren und Polymeren unterschiedlicher Kettenlängen, welche mit molekularem Formaldehyd und Formaldehyd-Hydrat im Gleichgewicht stehen. Sogenanntes Paraformaldehyd fällt hierbei als ein weißer, schlecht-löslicher Feststoff, aus der Lösung aus und stellt in der Regel ein Gemisch linearer Formaldehyd-Polymere mit n = 8 bis 100 Oxymethylen-Wiederholungseinheiten dar.

Ein Vorteil des erfindungsgemäßen Verfahrens liegt insbesondere darin, dass polymeres Formaldehyd bzw. sogenanntes Paraformaldehyd, welches kommerziell und kostengünstig erhältlich ist, direkt als Starterverbindung eingesetzt werden kann, ohne dass hierbei zusätzliche vorbereitende Schritte nötig sind. In einer vorteilhaften Ausführungsform der Erfindung wird daher Paraformaldehyd als Starterverbindung eingesetzt. Insbesondere lassen sich über das Molekulargewicht und die Endgruppenfunktionalität der polymeren Formaldehyd-Starterverbindung Polyoxymethylen-Blöcke mit definiertem Molgewicht und Funktionalität in das Produkt einbringen.

Vorteilhafterweise kann hierbei im erfindungsgemäßen Verfahren die Länge des Polyoxymethylen-Blocks einfach über das Molekulargewicht der eingesetzten Formaldehyd-Starterverbindung gesteuert werden. Vorzugsweise werden hierbei lineare Formaldehyd-Starterverbindungen der allgemeinen Formel HO-$(CH_2O)_n$-H, wobei n für eine ganze Zahl ≥ 2 steht, bevorzugt mit n= 2 bis 1000, besonders bevorzugt mit n = 2 bis 400 und ganz besonders bevorzugt mit n = 8 bis 100, mit zwei terminalen Hydroxylgruppen eingesetzt. Insbesondere können als Starterverbindung auch Gemische von polymeren Formaldehyd-Verbindungen der Formel HO-$(CH_2O)_n$-H mit jeweils unterschiedlichen Werten

für n eingesetzt werden. In einer vorteilhaften Ausführungsform enthalten die eingesetzten Gemische von polymeren Formaldehyd-Starterverbindungen der Formel $HO-(CH_2O)_n-H$ wenigstens 1 Gew-%, bevorzugt wenigstens 5 Gew.-% und besonders bevorzugt wenigstens 10 Gew.-% an polymeren Formaldehyd-Verbindungen mit $n \geq 20$.

Über das erfindungsgemäße Verfahren können insbesondere Polyoxymethylen-Blockcopolymere erhalten werden, welche eine A-B-A Blockstruktur umfassend einen inneren Polyoxymethylen-Block (B) und äußere oligomere Blöcke (A) aufweisen. Es ist erfindungsgemäß ebenso möglich, dass Formaldehyd-Starterverbindungen mit einer Hydroxy-Endgruppen-Funktionalität F > 2 eingesetzt werden, worüber sich folglich homologe Blockstrukturen $B(-A)_y$ mit einer Anzahl y >2 an äußeren oligomeren Blöcken (A), die entsprechend aus der Funktionalität der eingesetzten Formaldehyd-Starterverbindung resultiert, darstellen lassen. Ebenso ist es grundsätzlich möglich, dass Formaldehyd-Starterverbindungen mit einer Funktionalität F < 2 eingesetzt werden, dies können beispielweise auch lineare Formaldehyd-Starterverbindungen mit F = 1 sein, welche an einem Kettenende mit einer Schutzgruppe oder durch andere chemische Reste substituiert sind.

Ein Polyoxymethylen-Block im Sinne der Erfindung bezeichnet eine polymere Struktureinheit $-(CH_2-O-)_x$ wobei x für eine ganze Zahl $\geq 2$ steht, die mindestens eine an zwei Sauerstoffatome gebundene $CH_2$-Gruppe enthält, welche über mindestens eines der Sauerstoffatome mit weiteren Methylengruppen oder anderen polymeren Strukturen verbunden ist. Bevorzugt enthalten Polyoxymethylen-Blöcke $-(CH_2-O-)_x$ durchschnittlich $x \geq 2$ bis $x \leq 1000$, mehr bevorzugt durchschnittlich $x \geq 2$ bis $x \leq 400$ und besonders bevorzugt durchschnittlich $x \geq 8$ bis $x \leq 100$ Oxymethylen-Einheiten. Im Sinne der Erfindung werden unter einem Polyoxymethylen-Block auch solche Blöcke verstanden, welche geringe Anteile weiterer monomerer und/oder oligomerer Einheiten enthalten, im Allgemeinen weniger als 25 mol% bezogen auf die Gesamtmenge der im Block enthaltenen Monomereneinheiten.

Vorzugsweise stellen die äußeren oligomeren Blöcke (A) Polyoxyalkylen- oder Polyoxyalkylencarbonat-Blöcke dar, wobei als Polyoxyalkylen- oder Polyoxyalkylencarbonat-Blöcke im Sinne der Erfindung auch solche Blöcke verstanden werden, in welche (geringe) Anteile weiterer Comonomere, im Allgemeinen weniger als 50 mol-%, bevorzugt weniger als 25 mol-%, bezogen auf die Gesamtmenge aller im oligomeren Block vorliegenden Wiederholungseinheiten, einpolymerisiert sind.

Ein Polyoxyalkylencarbonat-Block im Sinne der Erfindung bezeichnet eine polymere Struktureinheit $-O[(C_2R^1R^2R^3R^4O)_x(CO_2)(C_2R^1R^2R^3R^4O)_y]_z-$, mit $x \geq 1$, $y \geq 0$ und $z \geq 1$, wobei $R^1$, $R^2$, $R^3$ und $R^4$ unabhängig voneinander Wasserstoff, ein gegebenenfalls zusätzliches Heteroatom wie Stickstoff, Sauerstoff, Silizium, Schwefel oder Phosphor enthaltender Alkyl- oder Arylrest sein und sich in verschiedenen Wiederholungseinheiten unterscheiden können. Der Begriff "Alkyl" umfasst allgemein im Zusammenhang der gesamten Erfindung Substituenten aus der Gruppe n-Alkyl wie Methyl, Ethyl oder Propyl, verzweigtes Alkyl und/oder Cycloalkyl. Der Begriff "Aryl" umfasst allgemein im Zusammenhang der gesamten Erfindung Substituenten aus der Gruppe einkernige Carbo- oder Heteroarylsubstituenten wie Phenyl und/oder mehrkernige Carbo- oder Heteroarylsubstituenten, die gegebenenfalls mit weiteren Alkylgruppen und/oder Heteroatomen wie Stickstoff, Sauerstoff, Silizium, Schwefel oder Phosphor substituiert sein können. Die Reste $R^1$, $R^2$, $R^3$ und/oder $R^4$ können innerhalb einer Wiederholungseinheit so miteinander verknüpft sein, dass sie zyklische Strukturen ausbilden, wie beispielsweise einen Cycloalkyl-Rest, der über zwei benachbarte Kohlenstoffatome in die Polymerkette eingebaut ist.

Es lassen sich durch das erfindungsgemäße Verfahren ausgehend von Formaldehyd-Starterverbindungen, welche als Gemisch unterschiedlicher Polymerkettenlängen vorliegen, wie beispielsweise Paraformaldehyd, Polyoxymethylen-Copolymere mit einem geringen Gehalt an Neben- und Zersetzungsprodukten und einer engen Molekulargewichtsverteilung erhalten. Ohne an eine bestimmte Theorie gebunden sein zu wollen, ist anzunehmen, dass während des Aktivierungsschrittes des DMC-Katalysators ebenfalls eine Konditionierung der Formaldehyd-Starterverbindung erfolgt, wobei eine Bildung von Neben- und Zersetzungsprodukten, (wie Formiaten, Methoxy-Derivaten, monomerem Formaldehyd), sowie die Defragmentierung des polymeren Formaldehyds zu kürzeren Kettenlängen verhindert und gleichzeitig eine hinreichende Aktivität und Selektivität des Katalysators erreicht wird. Hierbei wird die Formaldehyd-Starterverbindung, welche in thermisch und chemisch labiler und zumeist unlöslicher Halbacetal-Form vorliegt durch die Reaktion mit dem Alkylenoxid in eine thermisch und chemisch stabile Form überführt. Überraschenderweise lässt sich der Schritt der Aktivierung des DMC-Katalysators mit der Konditionierung des polymeren Formaldehyd-Starters kombinieren und besonders vorteilhaft bei unerwartet milden Temperaturen durchführen. Dergleichen war nicht zu erwarten, da DMC-Katalysatoren typischerweise deutlich höhere Temperaturen, beispielsweise von 130 °C, zur Aktivierung benötigen. Die Konditionierung der Formaldehyd-Starterverbindung in Gegenwart des DMC-Katalysators ermöglicht es, dass der Starter im darauffolgenden Polymerisationsschritt auch bei höheren Reaktionstemperaturen mit Alkylenoxiden und den gegebenenfalls weiteren Comonomeren umgesetzt werden kann, ohne dass es dann zu einer weiteren Defragmentierung und/oder der Bildung von Neben- und Zersetzungsprodukten kommt. Ein weiterer Vorteil ist, dass die konditionierte Formaldehyd-Starterverbindung zumeist nach der Konditionierung eine wesentlich höhere Löslichkeit aufweist, so dass nur geringe Mengen oder keine weiteren Lösungs- und/oder Suspensionsmittel benötigt werden.

Weiterhin lässt sich durch das erfindungsgemäße Verfahren sicherstellen, dass ein aktives DMC-Katalysatorsystem für die Polymerisation vorliegt und eine stetig voranschreitende Polymerisation unter kontinuierlicher Zugabe der Comonomere ein sicheres Verfahren sowie hohe Produktqualität sicherstellt.

Erfindungsgemäß erfolgt die Aktivierung des DMC-Katalysators daher in Gegenwart der polymeren Formaldehyd-Starterverbindung. Die Starterverbindung und der DMC-Katalysator können hierbei gegebenenfalls in einem Suspensionsmittel suspendiert sein. Ebenso ist es auch möglich eine weitere flüssige Starterverbindung ("Costarter") im Gemisch einzusetzen, wobei der DMC-Katalysator und die polymere Formaldehyd-Starterverbindung in dieser suspendiert sind.

Die Aktivierung des DMC-Katalysators erfolgt erfindungsgemäß bei einer Aktivierungstemperatur $T_{act}$ im Bereich von 20 bis 120 °C, bevorzugt bei 30 bis 120 °C, besonders bevorzugt bei 40 bis 100 °C und ganz besonders bevorzugt bei 60 bis 100 °C.

Unter "Aktivierung" des DMC-Katalysators ist ein Schritt zu verstehen, bei dem eine Teilmenge Alkylenoxid bei der spezifischen Aktivierungstemperatur zur DMC-Katalysator-Suspension zugegeben wird und dann die Zugabe des Alkylenoxids unterbrochen wird, wobei aufgrund einer folgenden exothermen chemischen Reaktion eine Wärmeentwicklung, die zu einer Temperaturspitze ("Hotspot") führen kann, sowie aufgrund der Umsetzung von Alkylenoxid ein Druckabfall im Reaktor beobachtet wird.

Für das erfindungsgemäße Verfahren geeignete DMC-Katalysatoren zur Verwendung in der Homopolymerisation von Alkylenoxiden sind im Prinzip aus dem Stand der Technik bekannt (siehe z.B. US-A 3 404 109, US-A 3 829 505, US-A 3 941 849 und US-A 5 158 922). DMC-Katalysatoren, die z.B. in US-A 5 470 813, EP-A 700 949, EP-A 743 093, EP-A 761 708, WO 97/40086, WO 98/16310 und WO 00/47649 beschrieben sind, besitzen eine sehr hohe Aktivität in der Polymerisation von Alkylenoxiden und ggf. der Copolymerisation von Alkylenoxiden mit geeigneten Comonomeren und ermöglichen die Herstellung von Polyoxymethylen-Copolymeren bei sehr geringen Katalysatorkonzentrationen, so dass eine Abtrennung des Katalysators aus dem fertigen Produkt i. a. nicht mehr erforderlich ist. Ein typisches Beispiel sind die in EP-A 700 949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch einen Polyether mit einem zahlenmittlerem Molekulargewicht größer als 500 g/mol enthalten.

Die Konzentration an eingesetztem DMC-Katalysator beträgt 10 bis 10000 ppm, besonders bevorzugt 20 bis 5000 ppm und höchst bevorzugt 50 bis 2000 ppm, bezogen auf die Masse des herzustellenden Polyoxymethylen-Block-Copolymers. Je nach Anforderungsprofil der nachgeschalteten Anwendung kann der DMC-Katalysator im Produkt belassen oder (teilweise) abgetrennt werden. Die (teilweise) Abtrennung des DMC-Katalysators kann beispielsweise durch Behandlung mit Adsorbentien und/oder Filtration erfolgen. Verfahren zur Abtrennung von DMC-Katalysatoren sind beispielsweise beschrieben in US-A-4,987,271, DE-A-3132258, EP-A-0 406 440, US-A-5,391,722, US-A-5,099,075, US-A-4,721,818, US-A-4,877,906 und EP-A-0 385 619.

Als Epoxid (Alkylenoxid) werden für die Herstellung der Polyoxymethylen-Blockcopolymere Verbindungen der allgemeinen Formel (I):

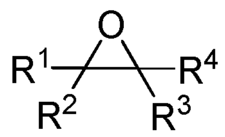

(I)

eingesetzt, wobei $R^1$, $R^2$, $R^3$ und $R^4$ unabhängig voneinander für Wasserstoff oder einen gegebenenfalls zusätzliche Heteroatome wie Stickstoff, Sauerstoff, Silizium, Schwefel oder Phosphor enthaltenden Alkyl- oder Arylrest stehen und ggf. so miteinander verknüpft sein können, dass sie zyklische Strukturen ausbilden, wie beispielsweise ein Cycloalkylenoxid.

Im Rahmen des erfindungsgemäßen Verfahrens können grundsätzlich solche Alkylenoxide eingesetzt werden, welche sich für die Polymerisation in Gegenwart eines DMC-Katalysators eignen. Werden verschiedene Alkylenoxide verwendet, so können diese entweder als Mischung oder nacheinander zudosiert werden. Bei letzterer Dosierweise können die Polyetherketten des hierüber erhaltenen Polyoxymethylen-Polyoxyalkylen-Blockcopolymers ihrerseits ebenfalls eine Blockstruktur aufweisen.

Allgemein können für das erfindungsgemäße Verfahren Alkylenoxide (Epoxide) mit 2-24 Kohlenstoffatomen eingesetzt werden. Bei den Alkylenoxiden mit 2-24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyoxysilane wie beispielsweise 3-Glycidyloxypropyltrimethoxysilan, 3-Glycidyioxypropyltriethoxysilan, 3-Glycidyloxypropyltripropoxysilan, 3-Glycidyloxypropyl-methyl-dimethoxysilan, 3-Glycidyloxypropyl-ethyldiethoxysilan, 3-Glycidyloxypropyltrlisopropoxysilan. Vorzugsweise ist das Epoxid der allgemeinen Formel (I) ein terminales Epoxid, wobei $R^1$, $R^2$ und $R^3$ für Wasserstoff stehen, und $R^4$ Wasserstoff, ein gegebenenfalls zusätzliche Heteroatome wie Stickstoff, Sauerstoff, Silizium, Schwefel oder Phosphor enthaltender Alkyl- oder Arylrest sein kann und sich in verschiedenen Wiederholungseinheiten unterscheiden kann. Vorzugsweise werden als Alkylenoxide Ethylenoxid und/oder Propylenoxid, insbesondere Propylenoxid eingesetzt.

Vorzugsweise wird das erfindungsgemäße Verfahren derart durchgeführt, dass sich an die Aktivierung des Katalysators und die Konditionierung der polymeren Formaldehyd-Starterverbindung in Schritt (β) ein Polymerisationsschritt (γ) unter Dosierung von einem oder mehreren Alkylenoxiden anschließt. Grundsätzlich kann das Verfahren aber auch nach Schritt (β) beendet werden, so dass die konditionierte polymere Formaldehyd-Starterverbindung dann das Endprodukt des Verfahrens darstellt. Diese weist im Allgemeinen durch die erfindungsgemäße Konditionierung eine hohe Stabilität auf und kann analog zu dem aus Schritt (γ) erhaltenen Polyoxymethylen-Blockcopolymer, wenn gewünscht, als OH-funktioneller Baustein für diverse Folgereaktionen eingesetzt werden.

In einer weiteren Ausführungsform des erfindungsgemäßen Verfahrens erfolgt die Polymerisation der Alkylenoxide in Gegenwart eines weiteren Comonomers. Als weitere Comonomere können beispielsweise alle Sauerstoffhaltigen cyclischen Verbindungen, insbesondere zyklische Ether, wie z.B. Oxetan, THF, Dioxan oder zyklische Acetale wie z.B. 1,3-Dioxolan oder 1,3-Dioxepan, zyklische Ester wie z.B. γ-Butyrolacton, γ-Valerolacton, ε-Caprolacton, oder zyklische Säureanhydride wie z.B. Maleinsäureanhydrid, Glutarsäureanhydrid oder Phthalsäureanhydrid sowie Kohlendioxid zum Einsatz kommen. Bevorzugt wird als Comonomer Kohlendioxid eingesetzt.

Die Dosierung weiterer Co-Monomere kann in Reinsubstanz, in Lösung oder als Mischung mit einem oder mehreren Alkylenoxiden erfolgen. Die Dosierung weiterer Co-Monomere kann ebenfalls parallel zu der Dosierung oder im Anschluss an die Dosierung der Alkylenoxide erfolgen.

In einer bevorzugten Ausführungsform des erfindungsgemäßen Verfahrens erfolgt neben der Anlagerung des oder der Alkylenoxide an die polymere Formaldehyd-Starterverbindung auch die Anlagerung von Kohlendioxid ($CO_2$) als weiteres Comonomer. Hierüber lassen sich Polyoxymethylen-Polyoxyalkylencarbonat-Copolymere herstellen. Diese weisen im Vergleich zu existierenden Produkten (zum Beispiel Polyetherpolyole im Polyurethanbereich beziehungsweise Polyoxymethylen-(Co-)Polymere im POM-Sektor) zusätzlich $CO_2$ als kostengünstiges und umweltfreundliches Comonomer auf. Da $CO_2$ u.a. ein Abfallprodukt der Energiegewinnung aus fossilen Rohstoffen ist und hier einer erneuten chemischen Verwertung zugeführt wird, ergeben sich durch den Einbau des $CO_2$ in die Polymerstrukturen neben ökonomischen auch ökologische Vorteile (günstige $CO_2$-Bilanz der Produktpolymere, etc.).

Polyoxymethylen-Polyoxyalkylencarbonat-Blockcopolymere im Sinne der Erfindung bezeichnen polymere Verbindungen, die mindestens einen Polyoxymethylen-Block sowie mindestens einen Polyoxyalkylencarbonat-Block enthalten. Polyoxymethylen-Polyoxyalkylencarbonat-Block-copolymere sind besonders interessant als Einsatzstoffe im Polyurethanbereich, sowie für Anwendungen im Polyoxymethylen (POM)-Sektor. Durch Veränderung des $CO_2$-Gehaltes können die physikalischen Eigenschaften auf die jeweilige Anwendung angepasst werden, wodurch neue Anwendungsgebiete für diese Blockcopolymere erschlossen werden können. Insbesondere lassen sich über das erfindungsgemäße Verfahren Polyoxymethylen-Polyoxyalkylencarbonat-Copolymere bereitstellen, wobei ein hoher Gehalt an eingebautem $CO_2$ erreicht wird, die Produkte eine vergleichsweise niedrige Polydispersität aufweisen und sehr wenige Neben- und Zersetzungsprodukte des polymeren Formaldehyds enthalten.

Die europäische Patentanmeldung EP13171772.0 offenbart ein Verfahren zur Herstellung von Polyoxyalkylencarbonat-Polyoxymethylen-Blockcopolymeren, wobei Polyoxyalkylencarbonate mit Zerewitinoff-aktiven H-Atomen als Starterverbindungen unter Aufpolymerisation von gasförmigem monomerem Formaldehyd umgesetzt werden. Allerdings können über dieses Verfahren keine Polyoxymethylen-Polyoxyalkylencarbonat-Blockcopolymere mit inneren Polyoxymethylen-Blöcken aufgebaut werden und die aus der Polymerisation mit monomerem Formaldehyd erhaltenen Produkte müssen in einem zusätzlichen Schritt mit Epoxiden, zyklischen Carbonsäure- oder Kohlensäureestern umgesetzt werden, um stabile Produkte mit terminalen Funktionalitäten zu erhalten.

Im Folgenden werden mehrere Varianten zur Durchführung des erfindungsgemäßen Verfahrens zur Herstellung von Polyoxymethylen-Blockcopolymeren durch Anlagerung von Alkylenoxiden und gegebenenfalls weiteren Comonomeren an polymere Formaldehyd-Starterverbindungen detailliert beschrieben. Die Darstellung ist lediglich beispielhaft und nicht als die vorliegende Erfindung beschränkend zu verstehen.

Beispielsweise ist das erfindungsgemäße Verfahren dadurch gekennzeichnet, dass im ersten Schritt (i)

(α) ein Suspensionsmittel oder eine polymere Formaldehyd-Starterverbindung vorgelegt und gegebenenfalls Wasser und/oder andere leicht flüchtige Verbindungen durch erhöhte Temperatur und/oder reduzierten Druck entfernt werden ("Trocknung"), wobei der DMC-Katalysator der polymeren Formaldehyd-Starterverbindung oder dem Suspensionsmittel vor oder nach der Trocknung zugesetzt wird,

(β) zur Aktivierung des DMC-Katalysators in Gegenwart der polymeren Formaldehyd-Starterverbindung eine Teilmenge (bezogen auf die Gesamtmenge der bei der Aktivierung und Polymerisation eingesetzten Menge an Alkylenoxiden) von einem oder mehreren Alkylenoxiden zu der aus Schritt (α) resultierenden Mischung zugesetzt wird, wobei diese Zugabe einer Teilmenge an Alkylenoxid gegebenenfalls in Gegenwart von weiteren Comonomeren, wie insbesondere $CO_2$, erfolgen kann, und wobei dann die aufgrund der folgenden exothermen chemischen Reaktion auftretende Temperaturspitze ("Hotspot") und/oder ein Druckabfall im Reaktor jeweils abgewartet wird, und wobei der Schritt (β) zur Aktivierung auch mehrfach erfolgen kann,
und im zweiten Schritt (ii)

(γ) ein oder mehrere Alkylenoxide und gegebenenfalls weitere Comonomere, insbesondere Kohlendioxid, zu der aus Schritt (β) resultierenden Mischung zugesetzt werden, wobei die in Schritt (γ) eingesetzten Alkylenoxide gleich oder verschieden sein können von den bei Schritt (β) eingesetzten Alkylenoxiden ("Polymerisation"),

wobei wenigstens in einem der Schritte (α) und (β) mindestens eine polymere Formaldehyd-Starterverbindung zugesetzt wird.

Die polymere Formaldehyd-Starterverbindung kann hierbei gemeinsam mit dem DMC-Katalysator und dem Suspensionsmittel in Schritt (α) vorgelegt werden, oder bevorzugt nach der Trocknung, spätestens in Schritt (β), zugegeben werden.

Die gegebenenfalls eingesetzten Suspensionsmittel enthalten im Allgemeinen keine H-funktionellen Gruppen. Als Suspensionsmittel sind alle polar-aprotischen, schwach polar-aprotischen und unpolar-aprotischen Lösungsmittel geeignet, die jeweils keine H-funktionellen Gruppen enthalten. Als Suspensionsmittel kann auch eine Mischung aus zwei oder mehreren dieser Suspensionsmittel eingesetzt werden. Beispielhaft seien an dieser Stelle folgende polar-aprotischen Suspensionsmittel genannt: 4-Methyl-2-oxo-1,3-dioxolan (im Folgenden auch als cyclisches Propylencarbonat oder cPC bezeichnet), 1,3-Dioxolan-2-on (im Folgenden auch als cyclisches Ethylencarbonat oder cEC bezeichnet), Aceton, Methylethylketon, Acetonitril, Nitromethan, Dimethylsulfoxid, Sulfolan, Dimethylformamid, Dimethylacetamid und N-Methylpyrrolidon. Zu der Gruppe der unpolar- und schwach polar-aprotischen Suspensionsmittel zählen z.B. Ether, wie z.B. Dioxan, Diethylether, Methyl-tert-Butylether und Tetrahydrofuran, Ester, wie z.B. Essigsäureethylester und Essigsäurebutylester, Kohlenwasserstoffe, wie z.B. Pentan, n-Hexan, Benzol und alkylierte Benzolderivate (z.B. Toluol, Xylol, Ethylbenzol) und chlorierte Kohlenwasserstoffe, wie z.B., Chloroform, Chlorbenzol, Dichlorbenzol und Tetrachlorkohlenstoff. Bevorzugt als Suspensionsmittel werden 4-Methyl-2-oxo-1,3-dioxolan, 1,3-Dioxolan-2-on, Toluol, Xylol, Ethylbenzol, Chlorbenzol und Dichlorbenzol sowie Mischungen zweier oder mehrerer dieser Suspensionsmittel eingesetzt, besonders bevorzugt ist 4-Methyl-2-oxo-1,3-dioxolan und 1,3-Dioxolan-2-on und Toluol oder eine Mischung aus 4-Methyl-2-oxo-1,3-dioxolan und 1,3-Dioxolan-2-on und/oder Toluol. Es ist ebenfalls auch möglich als Suspensionsmittel eine weitere Starterverbindung, welche unter den Reaktionsbedingungen in flüssiger Form vorliegt, im Gemisch mit der polymeren Formaldehyd-Starterverbindung einzusetzen.

Zu Schritt (α): (Trocknung)

Die Zugabe der einzelnen Komponenten in Schritt (α) kann gleichzeitig oder nacheinander in beliebiger Reihenfolge erfolgen.

Bevorzugt wird in Schritt (α) ein Suspensionsmittel, das keine H-funktionellen Gruppen enthält, im Reaktor vorgelegt. Anschließend wird die für die Polymerisation benötigte Menge an DMC-Katalysator, der bevorzugt nicht aktiviert ist, in den Reaktor gegeben. Die Reihenfolge der Zugabe ist dabei nicht entscheidend. Es kann auch erst der DMC-Katalysator und anschließend das Suspensionsmittel in den Reaktor gefüllt werden. Alternativ kann auch erst der DMC-Katalysator in dem Suspensionsmittel suspendiert und anschließend die Suspension in den Reaktor gefüllt werden. Durch das Suspensionsmittel wird eine ausreichende Wärmeaustauschfläche mit der Reaktorwand oder im Reaktor eingebauten Kühlelementen zur Verfügung gestellt, so dass die freigesetzte Reaktionswärme sehr gut abgeführt werden kann. Außerdem stellt das Suspensionsmittel bei einem Kühlungsausfall Wärmekapazität zur Verfügung, so dass die Temperatur in diesem Falle unterhalb der Zersetzungstemperatur des Reaktionsgemisches gehalten werden kann. Alternativ können auch in Schritt (α) ein Suspensionsmittel, das keine H-funktionellen Gruppen enthält, und zusätzlich eine Teilmenge der polymeren Formaldehyd-Starterverbindung sowie gegebenenfalls DMC-Katalysator im Reaktor vorgelegt werden, oder es können auch in Schritt (α) eine Teilmenge der polymeren Formaldehyd-Starterverbindung sowie gegebenenfalls DMC-Katalysator im Reaktor vorgelegt werden. Weiterhin können auch in Schritt (α) die Gesamtmenge der polymeren Formaldehyd-Starterverbindung sowie gegebenenfalls DMC-Katalysator im Reaktor vorgelegt werden.

Die polymere Formaldehyd-Starterverbindung kann hierbei grundsätzlich als Gemisch mit weiteren polymeren Formaldehyd-Starterverbindungen oder anderen H-funktionellen Starterverbindungen vorgelegt werden.

Das Verfahren kann derart durchgeführt werden, dass in Schritt (α) ein Suspensionsmittel, die polymere Formaldehyd-Starterverbindung und der DMC-Katalysator vorgelegt werden und gegebenenfalls Wasser und/oder andere leicht flüchtige Verbindungen durch erhöhte Temperatur und/oder reduzierten Druck entfernt werden ("Trocknung") oder in einer alternativen Ausführungsform der Schritt (α) derart durchgeführt wird, dass in einem Schritt (α1) ein Suspensionsmittel und der DMC-Katalysator vorgelegt und gegebenenfalls Wasser und/oder andere leicht flüchtige Verbindungen durch erhöhte Temperatur und/oder reduzierten Druck entfernt werden ("Trocknung") und in einem darauffolgenden Schritt (α2) zu dem Gemisch aus Schritt (α1) die Formaldehyd-Starterverbindung zugegeben wird. Die Zugabe der polymeren Formaldehyd-Starterverbindung kann nach Abkühlung der Reaktionsmischung aus Schritt (α1), insbesondere bei Raumtemperatur, erfolgen, oder es kann die Reaktionsmischung bereits auf die im darauffolgenden Schritt (β) vorherrschende Temperatur gebracht werden und die Zugabe bei dieser Temperatur erfolgen. Die Zugabe der Formaldehyd-Starterverbindung erfolgt im Allgemeinen unter inerten Bedingungen.

Der DMC-Katalysator wird vorzugsweise in einer Menge eingesetzt, so dass der Gehalt an DMC-Katalysator im resultierenden Reaktionsprodukt 10 bis 10000 ppm, besonders bevorzugt 20 bis 5000 ppm und höchst bevorzugt 50 bis 2000 ppm beträgt.

In einer bevorzugten Ausführungsform wird in das resultierende Gemisch aus Suspensionsmittel und DMC-Katalysator und/oder der polymeren Formaldehyd-Starterverbindung bei einer Temperatur von 90 bis 150°C, besonders bevorzugt von 100 bis 140°C Inertgas (beispielsweise Argon oder Stickstoff), ein Inertgas-Kohlendioxid-Gemisch oder Kohlendioxid eingeleitet und gleichzeitig ein reduzierter Druck (absolut) von 10 mbar bis 800 mbar, besonders bevorzugt von 50 mbar bis 200 mbar angelegt.

In einer alternativen bevorzugten Ausführungsform wird das resultierende Gemisch aus DMC-Katalysator mit Suspensionsmittel und/oder der polymeren Formaldehyd-Starterverbindung bei einer Temperatur von 90 bis 150°C, besonders bevorzugt von 100 bis 140°C mindestens einmal, vorzugsweise dreimal mit 1 bar bis 100 bar (absolut), besonders bevorzugt 3 bar bis 50 bar (absolut) eines Inertgases (beispielsweise Argon oder Stickstoff), eines Inertgas-Kohlendioxid-Gemisches oder Kohlendioxid beaufschlagt und jeweils anschließend der Überdruck auf ca. 1 bar bis 20 bar(absolut) reduziert.

Der DMC-Katalysator kann beispielweise in fester Form oder als Suspension in einem oder mehreren Suspensionsmittel(n) oder - falls die polymere Formaldehyd-Starterverbindung in einem flüssigen Aggregatszustand vorliegt - als Suspension in einer polymeren Formaldehyd-Starterverbindung zugegeben werden.

<u>Zu Schritt (β):</u> (Aktivierung)

Schritt (β) dient der Aktivierung des DMC-Katalysators. Dieser Schritt kann gegebenenfalls unter Inertgasatmosphäre, unter einer Atmosphäre aus Inertgas-Kohlendioxid-Gemisch oder unter einer Kohlendioxid-Atmosphäre durchgeführt werden. Als Aktivierung im Sinne dieser Erfindung wird ein Schritt bezeichnet, bei dem eine Teilmenge Alkylenoxid bei Temperaturen von 20 bis 120°C ("Aktivierungstemperatur") zur DMC-Katalysator-Suspension zugegeben wird und dann die Zugabe des Alkylenoxids unterbrochen wird, wobei aufgrund einer folgenden exothermen chemischen Reaktion eine Wärmeentwicklung, die zu einer Temperaturspitze ("Hotspot") führen kann, sowie aufgrund der Umsetzung von Alkylenoxid und gegebenenfalls $CO_2$ ein Druckabfall im Reaktor beobachtet wird.

In einer bevorzugten Ausführungsform beträgt die bei der Aktivierung in Schritt (β) eingesetzte Menge von einem oder mehreren Alkylenoxiden 2 bis 100 Stoffmengenäquivalente, bevorzugt 4 bis 50 Stoffmengenäquivalente, besonders bevorzugt 4,5 bis 25 Stoffmengenäquivalente bezogen auf die eingesetzte Stoffmenge an polymerer Formaldehyd-Starterverbindung, wobei das zahlenmittlere Molekulargewicht ($M_n$) der Formaldehyd-Starterverbindung bzw. der eingesetzten Gemische zugrunde gelegt wird. Das Alkylenoxid kann in einem Schritt oder schrittweise in mehreren Teilmengen zugegeben werden. Bevorzugt wird nach Zugabe einer Teilmenge an Alkylenoxid die Zugabe des Alkylenoxids bis zum Auftreten der Wärmeentwicklung unterbrochen und erst dann die nächste Teilmenge an Alkylenoxid zugegeben.

Für das erfindungsgemäße Verfahren hat sich weiterhin gezeigt, dass die Aktivierung (Schritt (β)) in Gegenwart der polymeren Formaldehyd-Starterverbindung zur Herstellung der Polyoxymethylen-Blockcopolymere vorteilhafterweise bei einer Aktivierungstemperatur $T_{act}$ von 20 bis 120 °C, bevorzugt bei 30 bis 120 °C, besonders bevorzugt bei 40 bis 100 °C und ganz besonders bevorzugt bei 60 bis 100 °C durchgeführt wird. Die Wärmeentwicklung durch die chemische Reaktion bei der Aktivierung des DMC-Katalysators führt erfindungsgemäß vorzugsweise nicht zu einem Überschreiten einer Temperatur von 120 °C im Reaktionsgefäß. Unterhalb von 20 °C läuft die Reaktion nur sehr langsam ab und eine Aktivierung des DMC-Katalysators dauert unverhältnismäßig lange oder findet ggf. nicht im gewünschten Umfang statt. Bei Temperaturen von 130 °C und höher steigt die Menge an unerwünschten Neben-/ Zersetzungsprodukten polymerer Formaldehyd-Starterverbindungen stark an. Es wird z.B. die Bildung von Formiat und Methoxy-Spuren beobachtet. Es hat sich weiterhin als Vorteil dieser Ausführungsform gezeigt, dass sich über eine genaue Abstimmung der Parameter in diesem Bereich ebenfalls die Eigenschaften des erhaltenen Polyoxymethylen-Blockcopolymers, insbesondere die Länge des Polyoxymethylen-Blocks, beeinflussen lassen.

Der Verfahrensschritt der Aktivierung ist die Zeitspanne von der Zugabe der Teilmenge an Alkylenoxid, gegebenenfalls in Gegenwart von $CO_2$, zum Reaktionsgemisch umfassend ein Suspensionsmittel, DMC-Katalysator und die Formaldehyd-Starterverbindung, bis zum Auftreten der Wärmeentwicklung (Exothermie). Gegebenenfalls kann die Teilmenge des Alkylenoxids in mehreren Einzelschritten, gegebenenfalls in Gegenwart von $CO_2$, zum Reaktionsgemisch gegeben werden und dann jeweils die Zugabe des Alkylenoxids unterbrochen werden. Im diesem Fall umfasst der Verfahrensschritt der Aktivierung die Zeitspanne von der Zugabe der ersten Teilmenge an Alkylenoxid, gegebenenfalls in Gegenwart von $CO_2$, zum Reaktionsgemisch bis zum Auftreten der Wärmeentwicklung nach Zugabe der letzten Teilmenge an Alkylenoxid. Im Allgemeinen kann dem Aktivierungsschritt ein Schritt zum Trocknen des DMC-Katalysators und ggf. der polymeren Formaldehyd-Starterverbindung bei erhöhter Temperatur und/oder reduziertem Druck, gegebenenfalls unter Durchleiten eines Inertgases durch die Reaktionsmischung, vorgelagert sein, wobei dieser Schritt der Trocknung nicht Teil des Aktivierungsschrittes im Sinne der vorliegenden Erfindung ist.

Die Dosierung eines oder mehrerer Alkylenoxide (und gegebenenfalls der weiteren Comonomere, insbesondere Kohlendioxid) kann prinzipiell in unterschiedlicher Weise erfolgen. Der Start der Dosierung kann aus dem Unterdruck heraus oder bei einem zuvor gewählten Vordruck erfolgen. Der Vordruck wird bevorzugt durch Einleiten eines Inertgases (wie beispielsweise Stickstoff oder Argon) oder von Kohlendioxid eingestellt, wobei der Druck (absolut) 5 mbar bis 100 bar, vorzugsweise 10 mbar bis 50 bar und bevorzugt 20 mbar bis 50 bar beträgt.

Eine alternative Ausführungsform ist auch eine zweistufige Aktivierung (Schritt β), wobei

(β-I) in einer ersten Aktivierungsstufe die Zugabe einer ersten Teilmenge von Alkylenoxid unter Inertgasatmosphäre erfolgt und

(β-II) in einer zweiten Aktivierungsstufe die Zugabe einer zweiten Teilmenge von Alkylenoxid unter Kohlendioxid-Atmosphäre erfolgt,

wobei die polymere Formaldehyd-Starterverbindung vor und nach Teilschritt (□□I) zugegeben werden kann.

<u>Zu Schritt (γ): (Polymerisation)</u>

Die Dosierung eines oder mehrerer Alkylenoxide kann simultan oder sequentiell über jeweils separate Dosierungen (Zugaben) oder über eine oder mehrere Dosierungen erfolgen. Werden mehrere Alkylenoxide zur Synthese der Polyoxymethylen-Blockcopolymere eingesetzt, so können die Alkylenoxide einzeln oder als Gemisch dosiert werden.

Für das erfindungsgemäße Verfahren hat sich gezeigt, dass die Polymerisation zur Herstellung des Polyetherblockes in den Polyoxymethylen-Polyoxyalkylen-Blockcopolymeren (Schritt (□)) vorteilhafterweise bei 50 bis 150 °C, bevorzugt bei 60 bis 145 °C, besonders bevorzugt bei 70 bis 140 °C und ganz besonders bevorzugt bei 90 bis 130 °C durchgeführt wird. Werden Temperaturen unterhalb von 50 °C eingestellt, läuft die Reaktion unverhältnismäßig langsam ab. Bei Temperaturen oberhalb von 150 °C steigt die Menge an unerwünschten Nebenprodukten stark an.

In einer weiteren Ausführungsform des erfindungsgemäßen Verfahrens erfolgt die Polymerisation in Gegenwart wenigstens eines Comonomers. Die Dosierung der weiteren Comonomere kann in Reinsubstanz, in Lösung oder sonst in jeglichen technisch-realisierbaren Formen erfolgen. Die Dosierung eines oder mehrerer Alkylenoxide und der Co-Monomere kann simultan oder sequentiell erfolgen, wobei die gesamte Menge an Co-Monomer auf einmal oder dosiert über die Reaktionszeit zugegeben werden kann. In einer bevorzugten Ausführungsform der Erfindung wird Kohlendioxid als Comonomer eindosiert. Die Dosierung eines oder mehrerer Alkylenoxide erfolgt simultan oder sequentiell zur Kohlendioxid-Dosierung. Über die Art der Dosierung der Alkylenoxide und der Comonomere, vorzugsweise Kohlendioxid, ist es möglich, Polyoxymethylen-Blockcopolymere mit statistischen, alternierenden, blockartigen oder gradientenartigen Polyether- und/oder Polyoxyalkylencarbonat -Blöcken zu synthetisieren.

Bei der Herstellung der Polyoxymethylen-Polyoxyalkylencarbonat-Blockcopolymere unter Copolymerisation von $CO_2$ als Comonomer wird vorzugsweise ein Überschuss an Kohlendioxid bezogen auf die zu erwartende bzw. abgeschätzte Menge an eingebautem Kohlendioxid im Polyoxyalkylencarbonatblock eingesetzt, da bedingt durch die Reaktionsträgheit von Kohlendioxid ein Überschuss von Kohlendioxid von Vorteil ist. Die Menge an Kohlendioxid kann über den Gesamtdruck bei den jeweiligen Reaktionsbedingungen festgelegt werden. Als Gesamtdruck (absolut) hat sich der Bereich von 0,01 bis 120 bar, bevorzugt 0,1 bis 110 bar, besonders bevorzugt von 1 bis 100 bar für die Copolymerisation zur Herstellung des Polyoxyalkylencarbonatblockes als vorteilhaft erwiesen. Für das erfindungsgemäße Verfahren hat sich weiterhin gezeigt, dass die Copolymerisation zur Herstellung des Polyoxyalkylencarbonatblockes vorteilhafterweise bei 50 bis 150 °C, bevorzugt bei 60 bis 145 °C, besonders bevorzugt bei 70 bis 140 °C und ganz

besonders bevorzugt bei 90 bis 130 °C durchgeführt wird. Werden Temperaturen unterhalb von 50 °C eingestellt, läuft die Reaktion unverhältnismäßig langsam ab. Bei Temperaturen oberhalb von 150 °C steigt die Menge an unerwünschten Nebenprodukten stark an. Weiterhin ist zu beachten, dass das $CO_2$ bei der Wahl von Druck und Temperatur vom gasförmigen Zustand möglichst in den flüssigen und/oder überkritischen flüssigen Zustand übergeht. $CO_2$ kann jedoch auch als Feststoff in den Reaktor gegeben werden und dann unter den gewählten Reaktionsbedingungen in den flüssigen und/oder überkritischen flüssigen Zustand übergehen.

Kohlendioxid kann in gasförmigem, festem, flüssigem oder überkritischem Zustand, bevorzugt im gasförmigen oder festen, besonders bevorzugt im gasförmigen Zustand eingesetzt werden. Bei Einsatz von Kohlendioxid im gasförmigen Zustand wird ein Kohlendioxid-Partialdruck von 1 bis 73,8 bar, bevorzugt von 1 bis 60 bar, besonders bevorzugt von 5 bis 50 bar gewählt. Die Kombination aus Druck und Temperatur wird bei Verwendung von gasförmigem Kohlendioxid derart gewählt, dass Kohlendioxid sich als Reinsubstanz unter den gewählten Reaktionsbedingungen im gasförmigen Zustand befindet. Die entsprechenden Bedingungen können anhand des Phasendiagrammes abgeleitet werden. Nach Einbringen von gasförmigem Kohlendioxid in den Reaktor löst sich dieses teilweise oder ganz in der Reaktionsmischung.

Die drei Schritte ($\alpha$), ($\beta$) und ($\gamma$) können in demselben Reaktor oder jeweils separat in unterschiedlichen Reaktoren durchgeführt werden. Besonders bevorzugte Reaktortypen für das erfindungsgemäße Verfahren sind Rührkessel, Rohrreaktor, und Schlaufenreaktor. Weiterhin können auch Extruder, Kneter, etc. als bevorzugte Reaktoren für das erfindungsgemäße Verfahren eingesetzt werden. Werden die Reaktionsschritte $\alpha$, $\beta$ und $\gamma$ in unterschiedlichen Reaktoren durchgeführt, kann für jeden Schritt ein unterschiedlicher Reaktortyp verwenden werden. Bei einer vollständig kontinuierlichen Reaktionsführung sind bevorzugt die einzelnen Schritte bzw. die Schritte ($\alpha$) und ($\beta$) von ($\gamma$) räumlich voneinander zu trennen, so dass eine getrennte Temperaturführung sowie eine geeignete Gaszufuhr und Anlegen von Unterdruck, Zugabe von polymerem Formaldehyd und Dosierung von Monomeren in den Einzelschritten erfindungsgemäß möglich ist.

Aufgrund ihrer thermischen und chemischen Stabilität lassen sich die erfindungsgemäßen Polyoxymethylen-Blockcopolymere, bzw. die aus dem Verfahren erhaltenen Produktgemische insbesondere destillativ aufarbeiten. Dabei kommen bevorzugt Dünnschichtverdampfer, Strangverdampfer und Stripp-Kolonnen sowie Kombinationen dieser zum Entfernen von Lösungs- bzw. Suspensionsmitteln, flüchtigen Bestandteilen und unreagierten Monomeren und/oder Oligomeren zum Einsatz. Jedoch sind hier auch prinzipiell alle anderen Apparaturen zur thermischen destillativen Aufarbeitung geeignet. Diese Art der Aufarbeitung kann kontinuierlich oder diskontinuierlich sowie parallel oder im Anschluss an die Reaktion erfolgen.

Ein weiterer Gegenstand der vorliegenden Erfindung sind Polyoxymethylen-Blockcopolymere, erhältlich durch das erfindungsgemäße Verfahren.

Das Molekulargewicht der erfindungsgemäßen Polyoxymethylen-Blockcopolymere ergibt sich insbesondere additiv aus dem Molekulargewicht der polymeren Formaldehyd-Starterverbindung und den aufpolymerisierten Blöcken. In einer Ausführungsform weisen die Polyoxymethylen-Blockcopolymere ein zahlenmittleres Molekulargewicht von $\leq$ 15000 g/mol, bevorzugt $\leq$ 9500 g/mol, besonders bevorzugt $\leq$ 6000 g/mol, ganz besonders bevorzugt $\leq$ 5000 g/mol, insbesondere von 200 g/mol bis 9500 g/mol, bevorzugt von 500 g/mol bis 5000 g/mol auf. Das zahlenmittlere Molekulargewicht lässt sich beispielsweise per Gel-Permeations-Chromatographie (GPC) gegen z.B. Polystyrol-Standards und/oder über experimentell ermittelte Hydroxylzahlen (OH#) bestimmen.

Die über das erfindungsgemäße Verfahren erhältlichen Polyoxymethylen-Blockcopolymere besitzen eine Blockstruktur umfassend einen inneren Polyoxymethylen-Block (B), umfassend wenigstens zwei und höchstens 1000 Oxymethyleneinheiten, bevorzugt wenigstens 2 und höchstens 400 Oxymethyleneinheiten, besonders bevorzugt von 8 bis 200, und ganz besonders bevorzugt wenigstens 8 und höchstens 100 Oxymethyleneinheiten, sowie wenigstens einen äußeren oligomeren Block (A), welcher bevorzugt einen Anteil von wenigstens 25 mol%, besonders bevorzugt wenigstens 50 mol-% Polyoxyalkyleneinheiten, bezogen auf die Gesamtmenge aller Oligomereneinheiten in diesem Block, umfasst. Die Anzahl an äußeren oligomeren Blöcken (A) resultiert entsprechend aus der Funktionalität der eingesetzten Formaldehyd-Starterverbindung. Vorzugsweise besteht das Polyoxymethylen-Polyoxyalkylen-Blockcopolymer ausschließlich aus den Blöcken A und B. In einer vorteilhaften Ausführungsform ist der äußere oligomere Block ein Polyoxyalkylenblock, besonders bevorzugt ein Polyoxyalkylencarbonatblock.

Die erfindungsgemäßen Polyoxymethylen-Blockcopolymere besitzen vorzugsweise terminale Hydroxy-Gruppen und haben vorzugsweise eine Funktionalität $F \geq 2$ (Anzahl an Hydroxy-Gruppen pro Molekül).

In einer weiteren Ausführungsform der Polyoxymethylen-Block-Copolymere haben diese eine monomodale Molekulargewichtsverteilung und einen Polydispersitätsindex (PDI) von $\leq$ 2,5, bevorzugt $\leq$ 2,2.

Die nach dem erfindungsgemäßen Verfahren erhältlichen Polyoxymethylen-Block-Copolymere enthalten vorzugsweise weniger als 2 Gew.-%, insbesondere weniger als 1 Gew.-% bezogen auf die Gesamtmasse des erhaltenen Polyoxymethylen-Block-Copolymers, Formiat und/oder Methoxy-Verunreinigungen.

Gegenstand der Erfindung ist ebenfalls ein Polyoxymethylen-Polyoxyalkylencarbonat-Block-Copolymer umfassend einen inneren Polyoxymethylen-Block ("Starter")sowie wenigstens einen äußeren Polyoxyalkylencarbonatblock

gemäß Formel (II)

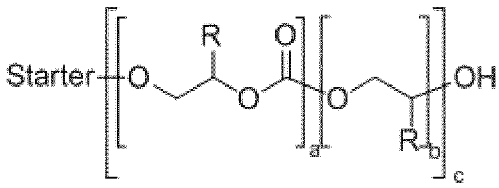

(II)

wobei R für einen organischen Rest wie Alkyl, Alkylaryl, Arylalkyl oder Aryl steht, der jeweils auch Heteroatome wie beispielsweise O, S, Si usw. enthalten kann,

und wobei a, b, und c für eine ganzzahlige Zahl stehen und R sich in verschiedenen Wiederholungseinheiten unterscheiden kann,

und wobei die Struktureinheit "Starter" einen aus der polymeren Formaldehyd-Starterverbindung hervorgehenden Polyoxymethylen-Block darstellt,

und wobei das hier im Schema (II) gezeigte Produkt für das Polyoxymethylen-Polyoxyalkylencarbonat-Block-Copolymer lediglich so verstanden werden soll, dass sich Blöcke mit der gezeigten Struktur im erhaltenen Polyoxymethylen-Polyoxyalkylencarbonat-Block-Copolymer prinzipiell wiederfinden können, die Reihenfolge, Anzahl und Länge der Blöcke sowie die OH-Funktionalität des "Starters" aber variieren kann und nicht auf das in Schema (II) gezeigte Polyoxymethylen-Polyoxyalkylencarbonat-Block-Copolymer beschränkt ist.

Die nach dem erfindungsgemäßen Verfahren erhältlichen Polyoxymethylen-Blockcopolymere weisen im Allgemeinen einen geringen Gehalt an Neben- und Zersetzungsprodukten, wie Formiat, Methoxy-Spuren, monomeres und oligomeres Formaldehyd, Restmonomere auf und können problemlos verarbeitet werden, insbesondere durch Umsetzung mit Di-, Tri und/oder Polyisocyanaten zu Polyurethanen, Isocyanat-funktionalisierten Polyurethan-Präpolymeren oder Polyisocyanuraten, insbesondere Polyurethan-Thermoplasten, Polyurethan-Beschichtungen, - Fasern, -Elastomeren, -Klebstoffen sowie insbesondere auch Polyurethan-Schaumstoffen eingeschlossen Weich- (wie beispielsweise Polyurethan-Weichblockschaumstoffe und Polyurethan-Weichformschaumstoffe) und Hartschäume. Für Polyurethananwendungen werden vorzugsweise Polyoxymethylen-Blockcopolymere eingesetzt, welche eine Funktionalität von mindestens 2 aufweisen. Des Weiteren können die nach dem erfindungsgemäßen Verfahren erhältlichen Polyoxymethylen-Blockcopolymere in Anwendungen wie Wasch- und Reinigungsmittelformulierungen, Klebstoffen, Farben, Lacken, Funktionsfluiden, Bohrflüssigkeiten, Kraftstoffadditiven, ionischen und nicht-ionischen Tensiden, Schmiermitteln, Prozesschemikalien für die Papier- oder Textilherstellung oder kosmetischen / medizinischen Formulierungen verwendet werden. Dem Fachmann ist bekannt, dass abhängig vom jeweiligen Anwendungsgebiet die zu verwendenden Polymere gewisse Stoffeigenschaften wie beispielsweise Molekulargewicht, Viskosität, Polydispersität, Funktionalität und/oder Hydroxylzahl (Anzahl an terminalen Hydroxy-Gruppen pro Molekül) erfüllen müssen.

Die Erfindung betrifft daher ebenfalls die Verwendung von erfindungsgemäßen Polyoxymethylen-Block-Copolymeren zur Herstellung von Polyurethan-Polymeren. In einer Ausführungsform dieser Verwendung sind die Polyurethan-Polymere Polyurethan-Weichschaumstoffe oder Polyurethan-Hartschaumstoffe. In einer weiteren Ausführungsform dieser Verwendung sind die Polyurethan-Polymere thermoplastische Polyurethan-Polymere.

Gegenstand der Erfindung ist daher ebenfalls ein Polyurethan-Polymer erhältlich durch Umsetzung eines Di-, Tri- und/oder Polyisocyanats mit wenigstens einem erfindungsgemäßen Polyoxymethylen-Block-Copolymer.

Gegenstand der Erfindung ist ebenfalls ein Polyurethan-Weichschaumstoff oder ein Polyurethan-Hartschaumstoff erhältlich durch Umsetzung eines Di-, Tri- und/oder Polyisocyanats mit wenigstens einem erfindungsgemäßen Polyoxymethylen-Block-Copolymer.

Erfindungsgemäß eingeschlossen ist auch die Verwendung von Polyoxymethylen-Block-Copolymeren gemäß der vorliegenden Erfindung zur Herstellung von Polyurethanen, Wasch- und Reinigungsmittelformulierungen, Bohrflüssigkeiten, Kraftstoffadditiven, ionischen und nicht-ionischen Tensiden, Schmiermitteln, Prozesschemikalien für die Papier- oder Textilherstellung oder kosmetischen Formulierungen.

## Beispiele

### Eingesetzte Verbindungen:

Es wurde Paraformaldehyd der Firma Ineos eingesetzt. Propylenoxid wurde von Sigma-Aldrich bezogen und ohne Aufreinigung eingesetzt. Als DMC-Katalysator wurde bei allen Beispielen DMC-Katalysator hergestellt gemäß Beispiel 6 in WO 01/80994 A1, enthaltend Zinkhexacyanocobaltat, tert.-Butanol und Polypropylenglykol mit einem zahlenmittleren Molekulargewicht von 1000 g/mol, eingesetzt.

**Beispiel 1: Herstellung eines Polyoxymethylen-Polyoxyalkylencarbonat-Blockcopolymers ($T_{act}$ = 40°C)**

In einen 300 mL Druckreaktor mit Gas- und Flüssigkeitsdosierungseinrichtung wurden 65 mg nicht aktivierter DMC-Katalysator in 30 g Toluol suspendiert. Die Suspension wurde unter Rühren auf 130 °C erhitzt, 40 bar $N_2$ aufgepresst und anschließend der $N_2$-Druck auf 15 bar gesenkt. Das Aufpressen und Ablassen von $N_2$ wurde in gleicher Weise noch zweimal durchgeführt. Die so erhaltene Suspension wurde auf Raumtemperatur abgekühlt und der $N_2$-Druck abgelassen. Der Druckreaktor wurde nun geöffnet und 15 g Paraformaldehyd (pFA) zur vorliegenden Suspension gegeben. Nachdem der Reaktor verschlossen und unter Rühren (1200 U/min) auf 40 °C ($T_{act}$) erhitzt wurde, erfolgte die Zugabe von 15 g Propylenoxid (PO) mit einer Dosiergeschwindigkeit von 10 g/min. Der Beginn der Reaktion machte sich durch einen Druckabfall auf den Ausgangsdruck sowie eine leichte Temperaturerhöhung bemerkbar. Nach der erfolgten Aktivierung wurde bei einer Temperatur von 100 °C und 50 bar Kohlendioxid ($CO_2$) 75 g PO kontinuierlich mit einer Dosierrate von 1 g/min und unter Rühren (1200 U/min) zugegeben. Der Fortschritt der Reaktion wurde mittels $CO_2$-Verbrauch beobachtet, wobei durch kontinuierliche Nachdosierung der Druck bei 50 bar im Reaktor konstant gehalten wurde. Nach beendeter PO-Zugabe wurde bei 100 °C und Reaktionsdruck 2 h nachgerührt (1200 U/min). Anschließend wurde auf Raumtemperatur abgekühlt. Das Reaktionsgemisch wurde mittels NMR- und GPC-Analytik untersucht und die Ergebnisse sind in Tabelle 1 dargestellt.

Die Zusammensetzung des Polymers wurde mittels $^1$H-NMR (Firma Bruker, DPX 400, 400 MHz; Pulsprogramm zg30, Wartezeit D1: 10s, 64 Scans) bestimmt. Die Probe wurde jeweils in deuteriertem Chloroform gelöst. Die relevanten Resonanzen im $^1$H-NMR (bezogen auf TMS = 0 ppm) und die Zuordnung der Flächenintegrale (A) ergeben sich wie folgt:

- cyclisches Propylencarbonat (cPC), Nebenprodukt, mit Resonanz bei 4,5 ppm, Flächenintegral entspricht einem H-Atom;
- lineares Propylencarbonat im Polymer (lPC) mit Resonanzen bei 1,2 bis 1,4 ppm, Flächenintegral minus 3 H-Atome des monomeren Propylenoxids (PO) entspricht somit 3 H-Atomen;
- monomeres Propylenoxid (PO), welches nicht abreagiert vorliegt, mit Resonanz bei 2,4 bzw. 2,75 ppm, Flächenintegral entspricht jeweils einem H-Atom;
- Polypropylenoxid (PPO), PO-Homopolymer, mit Resonanzen bei 1,0 bis 1,2 ppm, Flächenintegral entspricht 3 H-Atomen;
- Poly- bzw. Paraformaldehyd (pFA) mit Resonanzen bei 4,6 bis 5,2 ppm, Flächenintegral minus je ein H-Atom des cyclischen Propylencarbonats (cPC) sowie des lineares Propylencarbonats (lPC) entspricht somit 2 H-Atomen;
- Formiat (HCOO), Nebenprodukt, mit Resonanz bei 8,1 ppm, Flächenintegral entspricht einem H-Atom;
- Methoxy (MeO), Nebenprodukt in Spuren, mit Resonanz bei 3,4 ppm.

Die Ermittlung der Molenanteile (x) der Reaktionsmischung erfolgt wie folgt:

- x(cPC) = A(4,5 ppm)
- x(lPC) = A(1,2-1,4 ppm) - (3 · x(PO))
- x(PO) = A(2,75 ppm) oder A(2,4 ppm)
- x(PPO) = A(1,0-1,2 ppm)/3
- x(pFA) = (A(4,6-5,2 ppm) - x(cPc) - x(lPC))/2
- x(HCOO) = A(8,1 ppm)

Die so ermittelte Zusammensetzung des Reaktionsgemischs wird anschließend in Gewichtsteile umgerechnet und auf 100 normiert. Zur Umrechnung der Gewichtsanteile werden folgende Molmassen (g/mol) verwendet: cPC und lPC = 102, PO und PPO = 58, pFA = 30 und HCOO = 45. Die Polymerzusammensetzung wird anhand der Anteile lPC, PPO und pFA berechnet und normiert, so dass auch hier die Angabe in Gewichtsteilen von 100 (Gew.-%) erfolgt. Der $CO_2$-Gehalt der Polymerstruktur wird zusätzlich in Gew.-% ausgewiesen: Anteil lPC · (44/(44 + 58)), wobei sich die

Faktoren jeweils aus den verwendeten Molmassen von $CO_2$ (Molmasse 44 g/mol) und der von Propylenoxid (Molmasse 58 g/mol) ergeben.

Das Gewichts- und Zahlenmittel des Molekulargewichts der entstandenen Polymere wurde mittels Gel-Permeations-Chromatographie (GPC) bestimmt. Es wurde in Anlehnung an DIN 55672-1 vorgegangen: "Gelpermeationschromatographie, Teil 1 - Tetrahydrofuran als Elutionsmittel". Dabei wurden Polystyrolproben bekannter Molmasse zur Kalibrierung verwendet.

Die Molmasse des pFA-Blocks im Produktpolymer wurde nach folgender Formel:

$$MW(pFA) = M_n(GPC) \cdot (\text{pFA-Anteil im Polymer, NMR})/100$$

berechnet, wobei $M_n$(GPC) das mittels GPC bestimmte Zahlenmittel $M_n$ darstellt. Angegeben wird die Polydispersität PDI für die Molmassenverteilung $M_w/M_n$.

**Beispiel 2: Herstellung eines Polyoxymethylen-Polyoxyalkylencarbonat-Blockcopolymers ($T_{act}$ = 60°C)**

Entsprechend des Beispiels 1 wurde ein Polyoxymethylen-Polyoxyalkylencarbonat-Polyol hergestellt, wobei die Temperatur der Katalysatoraktivierung bei 60 °C ($T_{act}$) eingestellt wurde. Die Ergebnisse sind in Tabelle 1 dargestellt.

**Beispiel 3: Herstellung eines Polyoxymethylen-Polyoxyalkylencarbonat-Blockcopolymers ($T_{act}$ = 80°C)**

Entsprechend des Beispiels 1 wurde ein Polyoxymethylen-Polyoxyalkylencarbonat-Polyol hergestellt, wobei die Temperatur der Katalysatoraktivierung bei 80 °C ($T_{act}$) eingestellt wurde. Die Ergebnisse sind in Tabelle 1 dargestellt.

**Beispiel 4: Herstellung eines Polyoxymethylen-Polyoxyalkylencarbonat-Blockcopolymers ($T_{act}$ = 100°C)**

Entsprechend des Beispiels 1 wurde ein Polyoxymethylen-Polyoxyalkylencarbonat-Polyol hergestellt, wobei die Temperatur der Katalysatoraktivierung bei 100 °C ($T_{act}$) eingestellt wurde. Die Ergebnisse sind in Tabelle 1 dargestellt.

**Beispiel 5: Herstellung eines Polyoxymethylen-Polyoxyalkylencarbonat-Blockcopolymers ($T_{act}$ = 130 °C) (Vergleichsbeispiel)**

Entsprechend des Beispiels 1 wurde ein Polyoxymethylen-Polyoxyalkylencarbonat-Polyol hergestellt, wobei die Temperatur der Katalysatoraktivierung bei 130 °C ($T_{act}$) eingestellt wurde. Die Ergebnisse sind in Tabelle 1 dargestellt.

**Beispiel 6: Herstellung eines Polyoxymethylen-Polyoxyalkylencarbonat-Blockcopolymers ($T_{act}$ = 150 °C) (Vergleichsbeispiel)**

Entsprechend des Beispiels 1 wurde ein Polyoxymethylen-Polyoxyalkylencarbonat-Polyol hergestellt, wobei die Temperatur der Katalysatoraktivierung bei 150 °C ($T_{act}$) eingestellt wurde. Die Ergebnisse sind in Tabelle 1 dargestellt.

**Beispiel 7: Herstellung eines Polyoxymethylen-Polyoxyalkylen-Blockcopolymers ($T_{act}$ = 70 °C)**

In einen 300 mL Druckreaktor mit Gas- und Flüssigkeitsdosierungseinrichtung wurden 65 mg nicht aktivierter DMC-Katalysator in 30 g Toluol suspendiert. Die Suspension wurde unter Rühren auf 130 °C erhitzt, 40 bar $N_2$ aufgepresst und anschließend der $N_2$-Druck auf 15 bar gesenkt. Das Aufpressen und Ablassen von $N_2$ wurde in gleicher Weise noch zweimal durchgeführt. Die so erhaltene Suspension wurde auf Raumtemperatur abgekühlt und der $N_2$-Druck abgelassen. Der Druckreaktor wurde nun geöffnet und 15 g pFA zur vorliegenden Suspension gegeben. Nachdem der Reaktor verschlossen und unter Rühren (1200 U/min) auf die 70 °C ($T_{act}$) erhitzt wurde, erfolgte die Zugabe von 15 g PO mit einer Dosiergeschwindigkeit von 10 g/min. Der Beginn der Reaktion machte sich durch einen Druckabfall auf den Ausgangsdruck sowie eine leichte Temperaturerhöhung bemerkbar. Nach der erfolgten Aktivierung wurde bei einer Temperatur von 100 °C 75 g PO kontinuierlich mit einer Dosierrate von 1 g/min und unter Rühren (1200 U/min) zugegeben. Nach beendeter PO-Zugabe wurde bei 100 °C 2 h nachgerührt (1200 U/min). Anschließend wurde auf Raumtemperatur abgekühlt. Das Reaktionsgemisch wurde mittels NMR-und GPC-Analytik untersucht. Die Ergebnisse sind in Tabelle 1 dargestellt.

**Beispiel 8: Herstellung eines Polyoxymethylen-Polyoxyalkylencarbonat-Blockcopolymers ($T_{act}$ = 60 °C)**

In einen 300 mL Druckreaktor mit Gas- und Flüssigkeitsdosierungseinrichtung wurden 65 mg nicht aktivierter DMC-Katalysator in 30 g cPC (4-Methyl-2-oxo-1,3-dioxolan, cyclisches Propylencarbonat) suspendiert. Die Suspension wurde dann auf 130 °C aufgeheizt und über 30 min wurde mit 2,5 L/h Stickstoff eingeleitet und gleichzeitig ein reduzierter Druck von 75-100 mbar angelegt. Die so erhaltene Suspension wurde auf Raumtemperatur abgekühlt. Der Druckreaktor wurde nun geöffnet und 15 g pFA zur vorliegenden Suspension gegeben. Nachdem der Reaktor verschlossen und unter Rühren (1200 U/min) auf 60 °C ($T_{act}$) erhitzt wurde, erfolgte die Zugabe von 15 g PO mit einer Dosiergeschwindigkeit von 10 g/min. Der Beginn der Reaktion machte sich durch einen Druckabfall auf den Ausgangsdruck sowie eine leichte Temperaturerhöhung bemerkbar. Anschließend wurde bei einer Temperatur von 100 °C und 50 bar $CO_2$ 75 g PO kontinuierlich mit einer Dosierrate von 1 g/min und unter Rühren (1200 U/min) zugegeben. Der Fortschritt der Reaktion wurde mittels $CO_2$-Verbrauch beobachtet, wobei durch kontinuierliche Nachdosierung der Druck bei 50 bar im Reaktor konstant gehalten wurde. Nach beendeter PO-Zugabe wurde bei 100 °C und Reaktionsdruck 2 h nachgerührt (1200 U/min). Anschließend wurde auf Raumtemperatur abgekühlt. Das Reaktionsgemisch wurde mittels NMR- und GPC-Analytik untersucht. Die Ergebnisse sind in Tabelle 1 dargestellt.

**Tabelle 1: Resultate der Polyoxymethylen-Blockcopolymer Herstellung**

| Nr | $T_{act}$ [°C] | Restgehalt PO[a] [Gew.-%] | IPC[b] [Gew.-%] | PPO[b] [Gew.-%] | pFA[b] [Gew.-%] | $CO_2$ im Polymer [Gew.-%] | Mn (GPC) [g/mol] | MW pFA-Block [g/mol] | Formiat[a] [Gew.-%] | Methoxy Spuren | PDI |
|---|---|---|---|---|---|---|---|---|---|---|---|
| 1 | 40 | 1,5 | 50,4 | 30,8 | 18,8 | 21,8 | 3518 | 661 | - | nein | 1,98 |
| 2 | 60 | 1,1 | 58,0 | 28,3 | 13,7 | 25,0 | 4733 | 648 | - | nein | 2,09 |
| 3 | 80 | 2,5 | 55,3 | 28,9 | 15,9 | 23,8 | 4189 | 660 | - | nein | 2,42 |
| 4 | 100 | 2,9 | 57,8 | 28,4 | 13,7 | 24,9 | 4257 | 585 | - | nein | 2,45 |
| 5* | 130 | 34,5 | 36,3 | 39,0 | 24,8 | 15,6 | 1200 | 297 | 2,4 | ja | k.A. |
| 6* | 150 | 57,9 | 21,6 | 46,5 | 31,9 | 9,3 | 1020 | 325 | 2,2 | ja | 3,98 |
| 7 | 70 | 0,5 | - | 86,8 | 13,2 | - | 4495 | 593 | - | nein | 1,19 |
| 8 | 60 | 0,8 | 49,1 | 33,7 | 17,3 | 21,2 | 3010 | 521 | - | nein | 1,52 |

* = Vergleichsbeispiel, a) im Reaktionsgemisch, b) im Polymer.

In Tabelle 1 sind die Resultate für die Herstellung der Polyoxymethylen-Blockcopolymere zusammengefasst. Über das erfindungsgemäße Verfahren lassen sich Produkte mit einem definierten Polyoxymethylen-Block (pFA-Block) und einer niedrigen Polydispersität erhalten, ohne dass eine Zersetzung oder Defragmentierung des als Starterverbindung eingesetzten Paraformaldehyds in kleinere Oligomere oder Monomere beobachtet wird. Weiterhin lassen sich über das erfindungsgemäße Verfahren durch Copolymerisation Polyoxymethylen-Polyoxyalkylencarbonat-Blockcopolymere erhalten, wobei nahezu vollständige Umsätze des Alkylenoxids bei gleichzeitig hohen $CO_2$-Einbauraten erreicht werden. Die Vergleichsbeispiele 5 und 6 zeigen, dass bei höheren Temperaturen, welche im Bereich üblicherweise eingestellter Aktivierungstemperaturen für DMC-Katalysatoren liegen, eine Defragmentierung des polymeren Formaldehyd-Starters erfolgt und es zur Bildung von Nebenprodukten kommt.

## Patentansprüche

1. Verfahren zur Herstellung von Polyoxymethylen-Blockcopolymeren durch katalytische Anlagerung von Alkylenoxiden sowie gegebenenfalls weiteren Comonomeren an wenigstens eine polymere Formaldehyd-Starterverbindung, welche wenigstens eine terminale Hydroxylgruppe aufweist, in Gegenwart eines Doppelmetallcyanid (DMC)-Katalysators, wobei

   (i) in einem ersten Schritt der DMC-Katalysator in Gegenwart der polymeren Formaldehyd-Starterverbindung aktiviert wird, wobei zur Aktivierung des DMC-Katalysators eine Teilmenge (bezogen auf die Gesamtmenge der bei der Aktivierung und Polymerisation eingesetzten Menge an Alkylenoxiden) von einem oder mehreren Alkylenoxiden zugesetzt wird,
   (ii) in einem zweiten Schritt ein oder mehrere Alkylenoxide sowie gegebenenfalls weitere Comonomere zu der aus Schritt (i) resultierenden Mischung zugesetzt werden, wobei die in Schritt (ii) eingesetzten Alkylenoxide gleich oder verschieden sein können von den bei Schritt (i) eingesetzten Alkylenoxiden,

   **dadurch gekennzeichnet, dass** die Aktivierung des DMC-Katalysators im ersten Schritt (i) bei einer Aktivierungstemperatur ($T_{act}$) von 20 bis 120 °C erfolgt.

2. Verfahren gemäß Anspruch 1, wobei wenigstens eine offenkettige polymere Formaldehyd-Starterverbindung mit n Oxymethylen-Wiederholungseinheiten und einer Funktionalität F von 1 bis 10, insbesondere eine lineare Formaldehyd-Starterverbindung der allgemeinen Formel $HO-(CH_2O)_n-H$ mit zwei terminalen Hydroxylgruppen, eingesetzt wird, wobei n eine ganze Zahl von n = 2 bis 400, insbesondere von 8 bis 100, ist und wobei die Starterverbindung insbesondere auch als Gemisch von polymeren Formaldehyd-Starterverbindungen mit jeweils unterschiedlichen Werten für n vorliegen kann.

3. Verfahren gemäß Anspruch 1 oder 2, wobei Paraformaldehyd als polymere Formaldehyd-Starterverbindung eingesetzt wird.

4. Verfahren gemäß einem der vorstehenden Ansprüche, wobei die in Schritt (i) eingesetzte Menge von einem oder mehreren Alkylenoxiden 2 bis 100 Stoffmengenäquivalente bezogen auf die eingesetzte Stoffmenge an polymerer Formaldehyd-Starterverbindung beträgt.

5. Verfahren gemäß einem der vorstehenden Ansprüche, wobei in Schritt (ii) Kohlendioxid als Comonomer zugesetzt wird.

6. Verfahren gemäß einem der vorstehenden Ansprüche, wobei

   im ersten Schritt (i)

   ($\alpha$) ein Suspensionsmittel oder die polymere Formaldehyd-Starterverbindung vorgelegt und gegebenenfalls Wasser und/oder andere leicht flüchtige Verbindungen durch erhöhte Temperatur und/oder reduzierten Druck entfernt werden, wobei der DMC-Katalysator der polymeren Formaldehyd-Starterverbindung oder dem Suspensionsmittel vor oder nach der Trocknung zugesetzt wird,
   ($\beta$) zur Aktivierung des DMC-Katalysators in Gegenwart der polymeren Formaldehyd-Starterverbindung eine Teilmenge (bezogen auf die Gesamtmenge der bei der Aktivierung und Polymerisation eingesetzten Menge an Alkylenoxiden) von einem oder mehreren Alkylenoxiden zu der aus Schritt ($\alpha$) resultierenden Mischung zugesetzt wird, wobei diese Zugabe einer Teilmenge an Alkylenoxid gegebenenfalls in Gegenwart

von weiteren Comonomeren, wie insbesondere $CO_2$, erfolgen kann, und wobei dann die aufgrund der folgenden exothermen chemischen Reaktion auftretende Temperaturspitze ("Hotspot") und/oder ein Druckabfall im Reaktor jeweils abgewartet wird, und wobei der Schritt (β) zur Aktivierung auch mehrfach erfolgen kann,

und im zweiten Schritt (ii)

(γ) ein oder mehrere Alkylenoxide und gegebenenfalls weitere Comonomere, insbesondere Kohlendioxid, zu der aus Schritt (β) resultierenden Mischung zugesetzt werden, wobei die in Schritt (γ) eingesetzten Alkylenoxide gleich oder verschieden sein können von den bei Schritt (β) eingesetzten Alkylenoxiden,

wobei wenigstens in einem der Schritte (α) und (β) mindestens eine polymere Formaldehyd-Starterverbindung zugesetzt wird.

**7.** Verfahren gemäß Anspruch 6, wobei die Polymerisation in Schritt (γ) bei einer Temperatur von 70 bis 140 °C durchgeführt wird.

**8.** Verfahren gemäß Anspruch 6 oder 7, wobei in Schritt (α) als Suspensionsmittel mindestens eine Verbindung ausgewählt aus der Gruppe bestehend aus 4-Methyl-2-oxo-1,3-dioxolan, 1,3-Dioxolan-2-on, Aceton, Methylethylketon, Acetonitril, Nitromethan, Dimethylsulfoxid, Sulfolan, Dimethylformamid, Dimethylacetamid, N-Methylpyrrolidon, Dioxan, Diethylether, Methyl-tert-Butylether, Tetrahydrofuran, Essigsäureethylester, Essigsäurebutylester, Pentan, n-Hexan, Benzol, Toluol, Xylol, Ethylbenzol, Chloroform, Chlorbenzol, Dichlorbenzol und Tetrachlorkohlenstoff eingesetzt wird.

**9.** Verfahren gemäß einem der Ansprüche 6 bis 8, wobei in Schritt (α)

(α1) ein Suspensionsmittel und der DMC-Katalysator vorgelegt und Wasser und/oder andere leicht flüchtige Verbindungen dadurch entfernt werden, dass bei einer Temperatur von 90 bis 150°C mindestens einmal, vorzugsweise dreimal mit 1 bar bis 100 bar (absolut) eines Inertgases beaufschlagt wird und jeweils anschließend der Überdruck auf ca. 1 bar bis 20 bar (absolut) reduziert wird
und in einem darauffolgenden Schritt
(α2) zu dem Gemisch aus Schritt (α1) die Formaldehyd-Starterverbindung zugegeben wird.

**10.** Polyoxymethylen-Block-Copolymer, erhältlich nach einem Verfahren gemäß einem der Ansprüche 1 bis 9.

**11.** Polyoxymethylen-Block-Copolymer gemäß Anspruch 10 mit einem Polydispersitätsindex ≤ 2,5.

**12.** Polyoxymethylen-Polyoxyalkylencarbonat-Block-Copolymer umfassend einen inneren Polyoxymethylen-Block ("Starter") sowie wenigstens einen äußeren Polyoxyalkylencarbonatblock gemäß Formel (II)

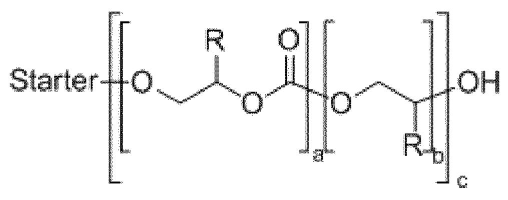

(II)

wobei R für einen organischen Rest wie Alkyl, Alkylaryl, Arylalkyl oder Aryl steht, der jeweils auch Heteroatome wie beispielsweise O, S, Si usw. enthalten kann,
und wobei a, b, und c für eine ganzzahlige Zahl stehen und R sich in verschiedenen Wiederholungseinheiten unterscheiden kann,
und wobei die Struktureinheit "Starter" einen aus der polymeren Formaldehyd-Starterverbindung hervorgehenden Polyoxymethylen-Block darstellt.

**13.** Polyurethan-Polymer erhältlich durch Umsetzung eines Di-, Tri- und/oder Polyisocyanats mit wenigstens einem Polyoxymethylen-Block-Copolymer gemäß einem der Ansprüche 10 bis 12.

**14.** Polyurethan-Weichschaumstoff oder Polyurethan-Hartschaumstoff erhältlich durch Umsetzung eines Di-, Tri- und/oder Polyisocyanats mit wenigstens einem Polyoxymethylen-Block-Copolymer gemäß einem der Ansprüche 10 bis 12.

**15.** Verwendung von Polyoxymethylen-Block-Copolymeren gemäß einem oder mehreren der Ansprüche 10 bis 12 zur Herstellung von Polyurethanen, Wasch- und Reinigungsmittelformulierungen, Bohrflüssigkeiten, Kraftstoffadditiven, ionischen und nicht-ionischen Tensiden, Schmiermitteln, Prozesschemikalien für die Papier- oder Textilherstellung oder kosmetischen Formulierungen

**Claims**

**1.** Process for preparing polyoxymethylene block copolymers by catalytic addition of alkylene oxides and optionally further comonomers onto at least one polymeric formaldehyde starter compound having at least one terminal hydroxyl group, in the presence of a double metal cyanide (DMC) catalyst, wherein

(i) in a first step the DMC catalyst is activated in the presence of the polymeric formaldehyde starter compound, activation of the DMC catalyst being accomplished by adding a portion (based on the totality of the amount of alkylene oxides used in the activation and polymerization) of one or more alkylene oxides,
(ii) in a second step one or more alkylene oxides and optionally further comonomers are added to the mixture resulting from step (i), where the alkylene oxides used in step (ii) may be the same as or different than the alkylene oxides used in step (i),

**characterized in that** the activation of the DMC catalyst in the first step (i) is effected at an activation temperature ($T_{act}$) of 20 to 120°C.

**2.** Process according to Claim 1, wherein at least one open-chain polymeric formaldehyde starter compound having n repeat oxymethylene units and a functionality F of 1 to 10, especially a linear formaldehyde starter compound of the general formula $HO-(CH_2O)_n-H$ having two terminal hydroxyl groups, is used, where n is an integer of n = 2 to 400, especially 8 to 100, and wherein the starter compound may especially also take the form of a mixture of polymeric formaldehyde starter compounds each having different values of n.

**3.** Process according to Claim 1 or 2, wherein paraformaldehyde is used as polymeric formaldehyde starter compound.

**4.** Process according to any of the preceding claims, wherein the amount of one or more alkylene oxides used in step (i) is 2 to 100 molar equivalents based on the amount of polymeric formaldehyde starter compound used.

**5.** Process according to any of the preceding claims, wherein carbon dioxide is added as comonomer in step (ii) .

**6.** Process according to any of the preceding claims, wherein

in the first step (i)

($\alpha$) a suspension medium or the polymeric formaldehyde starter compound is initially charged and, optionally, water and/or other volatile compounds are removed by means of elevated temperature and/or reduced pressure, the DMC catalyst being added to the polymeric formaldehyde starter compound or to the suspension medium before or after the drying,
($\beta$) activation of the DMC catalyst in the presence of the polymeric formaldehyde starter compound is accomplished by adding a portion (based on the entirety of the amount of alkylene oxides used in the activation and polymerization) of one or more alkylene oxides to the mixture resulting from step ($\alpha$), where this portion of alkylene oxide may optionally be added in the presence of further comonomers such as $CO_2$ in particular and where the temperature spike ("hotspot") which occurs due to the exothermic chemical reaction that follows and/or a pressure drop in the reactor is then awaited in each case, and where step ($\beta$) for activation may also be repeated,

and in the second step (ii)

($\gamma$) one or more alkylene oxides and optionally further comonomers, especially carbon dioxide, are added

to the mixture resulting from step (β), where the alkylene oxides used in step (γ) may be the same as or different than the alkylene oxides used in step (β),

wherein at least one polymeric formaldehyde starter compound is added at least in one of steps (α) and (β).

**7.** Process according to Claim 6, wherein the polymerization in step (γ) is conducted at a temperature of 70 to 140°C.

**8.** Process according to Claim 6 or 7, wherein the suspension medium used in step (α) is at least one compound selected from the group consisting of 4-methyl-2-oxo-1,3-dioxolane, 1,3-dioxolan-2-one, acetone, methyl ethyl ketone, acetonitrile, nitromethane, dimethyl sulfoxide, sulfolane, dimethylformamide, dimethylacetamide, N-methylpyrrolidone, dioxane, diethyl ether, methyl tert-butyl ether, tetrahydrofuran, ethyl acetate, butyl acetate, pentane, n-hexane, benzene, toluene, xylene, ethylbenzene, chloroform, chlorobenzene, dichlorobenzene and carbon tetrachloride.

**9.** Process according to any of Claims 6 to 8, wherein, in step (α),

(α1) a suspension medium and the DMC catalyst are initially charged and water and/or other volatile compounds are removed by, at a temperature of 90 to 150°C, injecting an inert gas at 1 bar to 100 bar (absolute) at least once, preferably three times, and each time subsequently reducing the elevated pressure to about 1 bar to 20 bar (absolute)
and, in a subsequent step,
(α2) the formaldehyde starter compound is added to the mixture from step (α1).

**10.** Polyoxymethylene block copolymer obtainable by a process according to any of Claims 1 to 9.

**11.** Polyoxymethylene block copolymer according to Claim 10, having a polydispersity index of ≤ 2.5.

**12.** Polyoxymethylene-polyoxyalkylene carbonate block copolymer comprising an inner polyoxymethylene block ("starter") and at least one outer polyoxyalkylene carbonate block of formula (II)

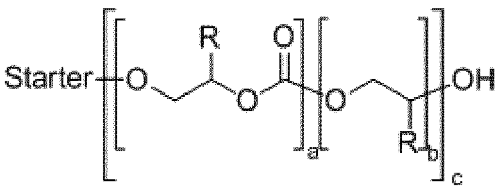

(II)

where R is an organic radical such as alkyl, alkylaryl, arylalkyl or aryl, each of which may also contain heteroatoms, for example O, S, Si, etc.,
and where a, b and c are each an integer and R may differ in different repeat units,
and where the structural unit "starter" represents a polyoxymethylene block deriving from the polymeric formaldehyde starter compound.

**13.** Polyurethane polymer obtainable by reacting a di-, tri- and/or polyisocyanate with at least one polyoxymethylene block copolymer according to any of Claims 10 to 12.

**14.** Flexible polyurethane foam or rigid polyurethane foam obtainable by reacting a di-, tri- and/or polyisocyanate with at least one polyoxymethylene block copolymer according to any of Claims 10 to 12.

**15.** Use of polyoxymethylene block copolymers according to one or more of Claims 10 to 12 for production of polyurethanes, washing and cleaning composition formulations, drilling fluids, fuel additives, ionic and nonionic surfactants, lubricants, process chemicals for papermaking or textile production, or cosmetic formulations.

## Revendications

**1.** Procédé de fabrication de copolymères séquencés de polyoxyméthylène par addition catalytique d'oxydes d'alkylène

et éventuellement d'autres comonomères sur au moins un composé démarreur formaldéhyde polymère, qui comprend au moins un groupe hydroxyle terminal, en présence d'un catalyseur à base de cyanure de métal double (DMC), selon lequel

(i) lors d'une première étape, le catalyseur DMC est activé en présence du composé démarreur formaldéhyde polymère, une partie (par rapport à la quantité totale d'oxydes d'alkylène utilisée lors de l'activation et de la polymérisation) d'un ou de plusieurs oxydes d'alkylène étant ajoutée pour l'activation du catalyseur DMC,
(ii) lors d'une deuxième étape, un ou plusieurs oxydes d'alkylène et éventuellement d'autres comonomères sont ajoutés au mélange résultant de l'étape (i), les oxydes d'alkylène utilisés à l'étape (ii) pouvant être identiques ou différents des oxydes d'alkylène utilisés à l'étape (i),

**caractérisé en ce que** l'activation du catalyseur DMC lors de la première étape (i) a lieu à une température d'activation ($T_{act}$) de 20 à 120 °C.

**2.** Procédé selon la revendication 1, dans lequel au moins un composé démarreur formaldéhyde polymère à chaîne ouverte contenant n unités de répétition oxyméthylène et ayant une fonctionnalité F de 1 à 10, notamment un composé démarreur formaldéhyde linéaire de formule générale $HO-(CH_2O)_n-H$ contenant deux groupes hydroxyle terminaux, est utilisé, n étant un nombre entier n = 2 à 400, notamment 8 à 100, et le composé démarreur pouvant notamment également se présenter sous la forme d'un mélange de composés démarreurs formaldéhyde polymères ayant chacun des valeurs différentes pour n.

**3.** Procédé selon la revendication 1 ou 2, dans lequel du paraformaldéhyde est utilisé en tant que composé démarreur formaldéhyde polymère.

**4.** Procédé selon l'une quelconque des revendications précédentes, dans lequel la quantité d'un ou de plusieurs oxydes d'alkylène utilisée à l'étape (i) est de 2 à 100 équivalents de quantité de matière, par rapport à la quantité de matière utilisée de composé démarreur formaldéhyde polymère.

**5.** Procédé selon l'une quelconque des revendications précédentes, dans lequel du dioxyde de carbone est ajouté en tant que comonomère à l'étape (ii).

**6.** Procédé selon l'une quelconque des revendications précédentes, dans lequel
lors de la première étape (i),
($\alpha$) un agent de suspension ou le composé démarreur formaldéhyde polymère est chargé, et de l'eau et/ou d'autres composés volatils sont éventuellement éliminés par une température élevée et/ou une pression réduite, le catalyseur DMC étant ajouté au composé démarreur formaldéhyde polymère ou à l'agent de suspension avant ou après le séchage,
($\beta$) pour l'activation du catalyseur DMC en présence du composé démarreur formaldéhyde polymère, une partie (par rapport à la quantité totale d'oxydes d'alkylène utilisée lors de l'activation et de la polymérisation) d'un ou de plusieurs oxydes d'alkylène est ajoutée au mélange résultant de l'étape ($\alpha$), cet ajout d'une partie de l'oxyde d'alkylène pouvant éventuellement avoir lieu en présence d'autres comonomères, tels que notamment du $CO_2$, et la pointe de température (« hotspot ») se produisant en raison de la réaction chimique exothermique qui a lieu et/ou une chute de pression étant alors à chaque fois attendues dans le réacteur, et l'étape ($\beta$) pour l'activation pouvant également avoir lieu à plusieurs reprises,
et lors de la deuxième étape (ii),
($\gamma$) un ou plusieurs oxydes d'alkylène et éventuellement d'autres comonomères, notamment du dioxyde de carbone, sont ajoutés au mélange résultant de l'étape ($\beta$), les oxydes d'alkylène utilisés à l'étape ($\gamma$) pouvant être identiques ou différents des oxydes d'alkylène utilisés à l'étape ($\beta$),
au moins un composé démarreur formaldéhyde polymère étant ajouté lors d'au moins une des étapes ($\alpha$) et ($\beta$).

**7.** Procédé selon la revendication 6, dans lequel la polymérisation à l'étape ($\gamma$) est réalisée à une température de 70 à 140 °C.

**8.** Procédé selon la revendication 6 ou 7, dans lequel au moins un composé choisi dans le groupe constitué par le 4-méthyl-2-oxo-1,3-dioxolane, la 1,3-dioxolan-2-one, l'acétone, la méthyléthylcétone, l'acétonitrile, le nitrométhane, le diméthylsulfoxyde, le sulfolane, le diméthylformamide, le diméthylacétamide, la N-méthyl-pyrrolidone, le dioxane, l'éther diéthylique, l'éther méthyl-tert-butylique, le tétrahydrofurane, l'ester éthylique de l'acide acétique, l'ester butylique de l'acide acétique, le pentane, le n-hexane, le benzène, le toluène, le xylène, l'éthylbenzène, le chloroforme, le chlorobenzène, le dichlorobenzène et le tétra-chlorocarbone est utilisé en tant qu'agent de suspension à l'étape ($\alpha$).

**9.** Procédé selon l'une quelconque des revendications 6 à 8, dans lequel, à l'étape ($\alpha$),
($\alpha$1) un agent de suspension et le catalyseur DMC sont chargés, et de l'eau et/ou d'autres composés volatils sont éliminés par chargement de 1 bar à 100 bar (absolu) d'un gaz inerte à une température de 90 à 150 °C à au moins une reprise, de préférence à trois reprises, puis à chaque fois réduction de la surpression à environ 1 bar à 20 bar (absolu),
et lors d'une étape ultérieure,
($\alpha$2) le composé démarreur formaldéhyde est ajouté au mélange de l'étape ($\alpha$1).

**10.** Copolymère séquencé de polyoxyméthylène, pouvant être obtenu par un procédé selon l'une quelconque des revendications 1 à 9.

**11.** Copolymère séquencé de polyoxyméthylène selon la revendication 10, ayant un indice de polydispersité $\leq$ 2,5.

**12.** Copolymère séquencé de polyoxyméthylène-carbonate de polyoxyalkylène comprenant une séquence polyoxyméthylène intérieure (« démarreur ») et au moins une séquence carbonate de polyoxyalkylène extérieure selon la formule (II)

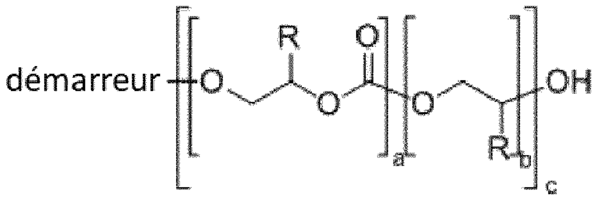

(II)

dans laquelle R représente un radical organique tel qu'un alkyle, un alkylaryle, un arylalkyle ou un aryle, qui peut à chaque fois également contenir des hétéroatomes tels que par exemple O, S, Si, etc.,
et dans laquelle a, b et c représentent un nombre entier et les R peuvent être différents dans différentes unités de répétition,
et dans laquelle l'unité structurale « démarreur » représente une séquence polyoxyméthylène provenant du composé démarreur formaldéhyde polymère.

**13.** Polymère de polyuréthane pouvant être obtenu par mise en réaction d'un di-, tri- et/ou polyisocyanate avec au moins un copolymère séquencé de polyoxyméthylène selon l'une quelconque des revendications 10 à 12.

**14.** Mousse souple de polyuréthane ou mousse dure de polyuréthane pouvant être obtenue par mise en réaction d'un di-, tri- et/ou polyisocyanate avec au moins un copolymère séquencé de polyoxyméthylène selon l'une quelconque des revendications 10 à 12.

**15.** Utilisation de copolymères séquencés de polyoxyméthylène selon une ou plusieurs des revendications 10 à 12 pour la fabrication de polyuréthanes, de formulations détergentes, de liquides de forage, d'additifs pour carburant, de tensioactifs ioniques et non ioniques, de lubrifiants, de produits chimiques de procédé pour la fabrication de papier ou de textiles, ou de formulations cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2007211082 A
- WO 2004096746 A1
- GB 807589 A
- EP 1418190 A1
- US 3754053 A
- US 3575930 A
- US 20020016395 A
- JP 4306215 A
- US 20060205915 A1
- EP 1870425 A1
- WO 2012091968 A1
- WO 1981001712 A1
- US 3436375 A
- JP 03263454 B
- JP 2928823 B
- US 3404109 A
- US 3829505 A
- US 3941849 A
- US 5158922 A
- US 5470813 A
- EP 700949 A
- EP 743093 A
- EP 761708 A
- WO 9740086 A
- WO 9816310 A
- WO 0047649 A
- US 4987271 A
- DE 3132258 A
- EP 0406440 A
- US 5391722 A
- US 5099075 A
- US 4721818 A
- US 4877906 A
- EP 0385619 A
- EP 13171772 A
- WO 0180994 A1


### In der Beschreibung aufgeführte Nicht-Patentliteratur


- **M. HAUBS.** Polyoxymethylenes, Ullmann's Encyclopedia of Industrial Chemistry. 2012
- *Bull. Chem. Soc. J.,* 1994, vol. 67, 2560-2566